(19) Europäisches Patentamt
European Patent Office
Office européen des brevets

(11) **EP 4 567 426 A1**

(12) **EUROPEAN PATENT APPLICATION**

(43) Date of publication:
**11.06.2025 Bulletin 2025/24**

(51) International Patent Classification (IPC):
**G01N 33/543** (2006.01)     **G01N 33/553** (2006.01)
**G01N 33/557** (2006.01)

(21) Application number: **23214385.9**

(22) Date of filing: **05.12.2023**

(52) Cooperative Patent Classification (CPC):
**G01N 33/54346; G01N 33/54373; G01N 33/553;
G01N 33/557**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
 • **Helmholtz Zentrum München - Deutsches
   Forschungszentrum für Gesundheit und Umwelt
   (GmbH)**
   **85764 Neuherberg (DE)**
 • **University Court of The University of St Andrews
   St Andrews
   Fife KY16 9AJ (GB)**

(72) Inventors:
 • **Cui, Jian
   85764 Neuherberg (DE)**

 • **Zhou, Jieying
   85764 Neuherberg (DE)**
 • **Ridderbeek, Korneel
   85764 Neuherberg (DE)**
 • **Sattler, Michael
   85764 Neuherberg (DE)**
 • **Kay, Euan
   St Andrews, KY16 9AJ (GB)**
 • **Falter-Braun, Pascal
   85764 Neuherberg (DE)**

(74) Representative: **Hepp Wenger Ryffel AG
Friedtalweg 5
9500 Wil (CH)**

Remarks:
The complete document including Reference
Table(s) and the Sequence Listing(s) can be
downloaded from the EPO website

(54) **A NANOPARTICLE, A SUSPENSION COMPRISING NANOPARTICLES, AND RELATED
METHOD**

(57)     The invention relates to a nanoparticle (1) having core (2), a first surface functionalization (4), and a second surface functionalization (5). The core (2) comprises or consists of a metal, preferably silver. An inorganic coating (3) is arranged around the core (2). The coating (3) preferably comprises or consists of gold. The first surface functionalization (4) is adapted to provide colloidal stability of the nanoparticle (1) in, preferably aqueous, solvents (19). The first functionalization (4) substantially prevents non-specific binding of macromolecules (55), in particular macromolecules including proteins, DNA, and/or RNA. The second surface functionalization (5) includes one of a binding site for a binding partner, preferably a binding site for a poly-histidine tag of a biomolecule (55), and a biomolecule, preferably a protein.

Fig. 1a

**Description**

The present invention relates to a nanoparticle, a suspension comprising nanoparticles, methods of synthesizing and functionalizing nanoparticles, and methods of measuring interactions between first and second biomolecules according to the preamble of the independent claims.

It is, in general, known in the prior art to use localized surface plasmon resonances (LSPR) to study interaction between molecular structures.

For example, US 2014/106469 A1 discloses a sensor for detecting the binding of molecules to membrane surfaces. The sensor comprises a nanoparticle coated with a continuous layer of silica, and having a ligand attached thereto, for detection of an analyte in a solution. The nanoparticle can be further coated with a continuous membrane, such as a lipid bilayer.

Flesch et al. (Anal. Bioanal. Chem. (2020), 412, 3413-3422, DOI : 10.1007/s00216-020-02551-6) discloses self-assembly of a functionalized gold nanoparticle monolayer on a glass substrate Site-specific reversible immobilization of proteins with a poly-histidine tag is accomplished by coating the monolayer with tris-nitrilotriacetic acid (trisNTA) PEG disulfide. LSPR spectroscopy detection is used to analyze cytokine-receptor interactions.

Hobbs et al. (Chem. Commun. (2016), 52, 9785-9788, DOI: 10.1039/C6CC05260F) discloses gold-plated silver nanoparticles with surface plasmon resonance (SPR) sensing response.

US 11,280,784 B2 discloses LSPR biosensing microfluidic or spot-plate device.

Murshid *et al.* discloses a synthesis protocol for silver nanoparticles with pentagonal symmetries (Murshid, N.; Keogh, D.; Kitaev, V. Optimized Synthetic Protocols for Preparation of Versatile Plasmonic Platform Based on Silver Nanoparticles with Pentagonal Symmetries. Part. Part. Syst. Charact. 2014, 31 (2), 178-189, DOI: 10.1002/ppsc.201300225).

Kitaev *et al.* discloses a synthesis for gold plating of silver nanoparticles (Mushid, N.; Gourevich, I.; Coombs, N.; Kitaev, V. Gold Plating of Silver Nanoparticles for Superior Stability and Preserved Plasmonic and Sensing Properties. Chem. Commun. 2013, 49 (97), 11355-11357, DOI: 10.1039/C3CC46075D).

However, devices and methods in the prior art are limited to the study of interaction between single analytes and sensing structures and/or require complicated microfluidic set-ups.

Thus, the object of the present invention is to overcome the drawbacks of the prior art, in particular to provide a versatile and simple approach to measuring interactions between different molecules in solution.

This and other objects are achieved by the nanoparticle, a suspension comprising nanoparticles, methods of synthesizing and functionalizing nanoparticles, and methods of measuring interactions between first and second biomolecules according to the characterizing portion of the independent claims of the invention.

The invention is directed to a nanoparticle having a core, an inorganic coating is arranged around the core, a first surface functionalization, and a second surface functionalization. The core comprises or consists of metal, preferably silver. The coating preferably comprises or consists of gold. The first surface functionalization is adapted to provide colloidal stability of the nanoparticle in solvents. Preferably, the solvent is an aqueous solvent, such as water, a biological buffer, or similar. The first surface functionalization substantially prevents non-specific binding of macromolecules. Macromolecules may, for example, include proteins, DNA, RNA, or similar biological macromolecules. The second surface functionalization may include a binding site for a binding partner, for example a binding partner for a poly-histidine tag (in the following referred to as "His-tag", or "His-tagged"). Additionally or alternatively, the first and/or second surface functionalization may comprise a biomolecule, for example a protein or a peptide.

The nanoparticle according to the invention may be used as a localized surface plasmon resonance (LSPR)-based sensor. LSPR-based sensors may respond optically to refractive-index changes within plasmonic "hotspots" located at the nanoparticle surface, typically at edges and tips. Given the small size of most proteins and their small refractive-index mismatch with water (~1.45 vs. 1.33), the optical sensing performance of the nanoparticle is a criterion for protein detection. This performance is commonly encapsulated by the optical sensing figure-of-merit (FOM), which is the ratio of the nanoparticle's refractive-index sensitivity and its spectral linewidth. In other words, the FOM is maximized with high refractive-index sensitivity and narrow spectral linewidth.

Both the refractive-index sensitivity and FOM may be determined by the nanoparticle material composition, geometry, and size. Among plasmonic nanoparticles at visible wavelengths, silver nanoparticles generally possess high refractive-index sensitivities and narrowest spectral linewidths, in particular silver decahedral nanoparticles. However, silver nanoparticles may have an insufficient chemical stability in biological solutions. Thus, coatings, for example with gold, may increase chemical stability, biocompatibility, and possibilities for surface functionalization.

In one aspect, the invention thus provides silver decahedral nanoparticles that possess excellent optical sensing properties. These nanoparticles are then coated with a thin layer of gold, improving chemical stability and compatibility with biological buffers, while largely maintaining their optical sensing performance. The nanoparticle surface may be adapted to reduce non-specific protein interactions and also enable specific, and modular, immobilization of one protein binding partner via a poly-histidine tag of recombinant proteins. Qualitative detection of specific protein-protein interactions, but also the quantification of binding parameters, may be done using these nanoparticles. This technique offers a straightforward, low-cost, and modular approach for measuring protein-protein interactions all within the solution phase and without complex instrumentation. The customizability of these nanoparticles enables extension of this approach to measurements of other biomolecular interactions, such as protein-RNA, or in complex biological solutions.

The inorganic coating may be thin in relation to the size of the nanoparticle/core and may typically have a thickness in the range of 1-10% of a diameter of the nanoparticle, e.g. approximately 1 nm. The thickness of the inorganic layer may also be uniform and cover the entire surface of the core. The material of the inorganic may be chosen to provide robustness against chemical stress and chemical resistance, functionalizability, biocompatibility, and plasmonic properties. The inorganic layer may be made of a different material than the core and may further be selected to minimize absorption of visible light. Gold has been shown to provide several of the above properties, thus being a very advantageous choice for as an inorganic coating. However, the person skilled in the art will appreciate that other materials providing one or several of the above properties may be suitable as well.

A functionalization may be understood as a surface modification, e.g. the addition of a capping agent, a ligand, a surface charge, or similar.

In some preferred embodiments, the first surface functionalization is a ligand which is adapted to bind to the surface of the nanoparticle, e.g. through a chemical group which reacts with the inorganic material. For example, thiolated ligands may bind to gold. The ligand may provide hydrophilicity such as to make the nanoparticles dispersable in water-based solvents.

A tag may be understood as a group on a biomolecule for linking, i.e. typically a functional group which can bind, such as Histidin.

Preferably, the nanoparticle has a characteristic size between 20 nm and 60 nm, particularly preferably between 30 nm and 45 nm.

A characteristic size may be a diameter, an average diameter, a largest size, a length, and/or a width of the nanoparticle. A size in this range may provide sufficient dispersability while retaining sensing properties. Particles known in the art for sensing are generally larger than 60 nm and may only have insufficient dispersability, which may be avoided by using particles with a size in the range of 20 nm to 60 nm. Smaller particles may also be less perturbative when sensing.

The nanoparticle may comprise or consist of flat facets delimited by edges which meet in a pyramid tip. The shape may have base surface from which the flat facets and edges extend. The base surface may be planar. The shape may be mirror-symmetrical with respect to the base surface. The nanoparticles may have a substantially decahedral shape, in particular a pentagonal bipyramid. A pentagonal bipyramid may have at least one flattened edge, forming a facet which is smaller than other pyramid side facets and separating the same.

In particular when the particles are small, it may be challenging to obtain sufficient scattering. Decahedral nanoparticles are particularly advantageous in that they provide sufficient scattering even at small sizes.

It will be understood by the skilled person that nanoparticles with a five-fold crystalline mismatch are known as decahedral nanoparticles. Furthermore, it will also be understood that the crystalline mismatch/flattened edge may create a sixth facet. Because such a sixth facet is typically much smaller than the remaining five, the overall shape may still be considered decahedral.

In general, the nanoparticle may have any shape comprising edges and/or corners, and/or flat facets, for example cubes, pyramids, bipyramids. Particularly preferably, the shape of the nanoparticle comprises or consists of flat facets delimited by edges which meet in a pyramid tip. The edges and/or corners are preferably sharp, though it will be understood by the skilled person that edges and corners of nanoparticles are not normally atomically sharp.

Typically, nanoparticles having shapes with edges provide higher sensitivity than nanoparticles without edges. Edges and corners may provide more confined "hotspots" of higher optical sensitivity".

The first surface functionalization may comprise a polymer, in particular polyethylene glycol or a derivative thereof. The first surface functionalization may be formed by a first ligand. The first ligand may have a molecular weight of less than 1.5 kDa, preferably 1 kDa.

The first surface functionalization may be formed by a peptide which may provide better pH and charge matching with biological systems. A suitable peptide for surface functionalization, in particular of nanoparticles with a gold surface, is, for example, disclosed in J. Am. Chem. Soc. 2012, 134, 13, 6000-6005 (DOI: 10.1021/ja3006868) and Langmuir 2014, 30, 7, 1864-1870 (DOI: 10.1021/la404980g).

The first ligand may be a molecule which contributes to the overall hydrodynamic radius by less than 2 nm. As a result, the first ligand does not take up much space vertically away from the surface and allows proteins, when bound to the second ligand, to be closer to the nanoparticle surface, which increases sensitivity.

The first ligand may be a thiolated polyethylene glycol or a derivative thereof. Particularly preferably, the first ligand is a methoxy polyethylene glycol or a derivative thereof, for example methoxy polyethylene glycol thiol.

The first ligand may be bound to the nanoparticle surface via the thiol end group.

The first ligand may, additionally or alternatively, comprise and preferably be terminated by a cysteine. In particular where the first ligand is a peptide, said peptide may be terminated by a cysteine which allows binding to surfaces such as gold surfaces due to its thiol group.

The second surface functionalization may include nitriloacetic acid chelated with a metal ion. Preferably, the metal ion is Ni(II) and/or Co(II). The second surface functionalization may be formed by a second ligand.

The second ligand may be thiolated alkane-polyethylene glycol-nitriloacetic acid chelated with Ni(II). Preferably, the second ligand may be methoxy polyethylene glycol thiol nitriloacetic acid chelated with Ni(II).

The second ligand may be bound to the surface of the nanoparticle via the thiol end group.

If the nanoparticle is functionalized with thiolated polyethylene glycol as a first ligand and thiolated alkane-polyethylene glycol-nitriloacetic acid chelated with Ni(II) as a second ligand, the ratio of first to second ligand is preferably 3:2.

In general, the ratio of first to second ligand may be in the range of 4:1 to 1:4, preferably 3:2 to 2:3.

The invention is further directed to a method of functionalizing colloidal nanoparticles. The method comprises providing nanoparticles, which may be colloidal nanoparticles with a gold coating. The nanoparticles may have a decahedral shape, for example a pentagonal bipyramid. The nanoparticles are suspended in a solvent. The solvent may be one of dimethyl sulfoxide, water, aqueous biological buffer, aqueous buffered saline, serum derived from blood, and/or cell extracts. A ligand mixture is added to the solvent. The ligand mixture preferably contains methoxy polyethylene glycol thiol and nitriloacetic acid. Preferably, the colloidal nanoparticles are incubated, i.e. shaken for approximately 12 hours at room temperature. The nanoparticles are subsequently washed at least once and preferably twice.

Functionalization may refer to the attachment of ligands to the surface of the colloidal nanoparticles. The method according to the invention may be used to functionalize any nanoparticle with a gold surface regardless of its shape. Nevertheless, the functionalization achieved with the method is particularly advantageous in nanoparticles having a shape comprising edges and/or corners, and/or flat facets, for example cubes, pyramids, bipyramids, because such nanoparticles with such shapes provide high sensitivity/LSPR response to biomolecules, e.g. proteins, binding to their surface. The extent to which such biomolecules may bind in suspension can be tuned, while avoiding excessive aggregation and precipitation, with the described functionalization.

A washing step typically includes dispersion of the nanoparticles in a liquid, for example 0.1 M Tris buffer containing 0.025 wt-% Tween 20, and separating the nanoparticles via centrifugation. The liquid may be disposed. If necessary, i.e. if the nanoparticles to be washed are suspended in a liquid, the nanoparticles may be separated by centrifugation before washing. After a washing step, the nanoparticles may be redispersed in any appropriate solvent to create a suspension.

The method may further comprise a step of incubating the nanoparticles in a solution comprising a metal salt. The metal salt may be $NiCl_2$. The solution may be an aqueous solution containing 50 $\mu$M metal salt. The nanoparticles may be washed at least once after the incubation step.

Incubation may be done for 1-5 h, preferably shorter than 2.5 h, at room temperature.

The nanoparticles may be separated by centrifugation and redispersed in the solution containing metal salt. Alternatively, a metal salt may be added to a suspension of nanoparticles.

The invention is further directed to a nanoparticle functionalized by the method disclosed herein.

The invention is further directed to a suspension comprising colloidal particles. The colloidal particles may be a plurality of any one, or several, of the nanoparticles disclosed herein. The suspension further comprises a solvent, preferably a biological buffer. The colloidal particles are suspended in the solvent.

The suspension may have a concentration of colloidal particles in the solvent, i.e. a number of particles per volume, between 0.01 nM and 20 nM. Preferably, the concentration is between 0.05 nM and 0.2 nM, which is particularly suitable for measurements using UV/VIS spectrophotometry. Alternatively, the concentration may be between 15 nM and 20 nM, for example if the measurements using photon-correlated Fourier spectroscopy (PCFS), or other photon correlation spectroscopies, or other solution-phase spectroscopic techniques are to be performed.

The invention is further directed to a method of determining at least one value representing an interaction between a first and a second molecule, for example an equilibrium dissociation constant. The method may determine several values. The first and/or second molecule may be a biomolecule, preferably a protein. Preferably, the value quantifies a magnitude of an interaction between the first and the second molecule, for example a kinetic association or dissociation rate constant. The method comprises providing a first suspension as described herein. Alternatively, colloidal particles may be provided which have a first molecule bound to their surface, in which case the first molecule may be a protein, a peptide, or another biomolecule which interacts with proteins.

Optionally, the first molecule is added to the first suspension. The first molecule may have a tag for specific binding to the binding site, such as to immobilize the first molecule to the binding site on the colloidal particles. Particularly preferably, a waiting step, e.g. of 10 min, is performed to allow the first molecule to bind to the binding site of the colloidal nanoparticles and reach an equilibrium.

A second molecule is added to the first suspension, resulting in a first concentration. The second molecule is added after the addition of the first molecule, if a step of adding a first molecule is performed. The second molecule may be a biomolecule, preferably a protein. A second waiting step may also be performed after the addition of the second protein to achieve an equilibrium in the binding between the first and second molecule. The second waiting step may be, for example, 10 min.

At least a first optical value is obtained after addition of the second molecule. The optical value may be, for example, the position of a LSPR peak and/or an energy and/or line shape of an LSPR spectrum measured by UV/VIS spectroscopy, by photon-correlated Fourier spectroscopy, or other photon correlation spectroscopies, or other solution-phase spectroscopic techniques. The optical value may be an amount of light scattered off by the nanoparticles, as disclosed, for example, in WO 2022/2014611 A1. If photon-correlation spectroscopy is used, the first concentration may be chosen higher, preferably by approximately a factor of 20, than if UV/VIS spectroscopy is used.

A change of the optical value, compared to a reference value, is then obtained. The reference value may be a measurement of the same optical value at a different time, in particular immediately preceding the addition of the second molecule and/or after addition of the first molecule. The change may be a shift in an LSPR peak.

The value representing an interaction between the first and second molecule may therefore be, for example, a shift of a LSPR peak which is caused by the interaction of a first protein with a second protein. Therefore, the interaction represented by the value may be a quantitative value, e.g. an equilibrium dissociation constant or a kinetic value such as a dissociation or association rate constant, or qualitative indication of whether a certain molecule binds to another molecule.

The step of measuring the first optical value may include measuring an optical extinction value of the suspension, preferably a recording of an extinction or absorption spectrum using a UV/VIS spectrophotometer, or other liquid-phase spectroscopy technique. The first optical value may be a position of an absorption peak corresponding to a localized surface plasmon resonance of the colloidal particle.

The method may include repeating the step of adding the second molecule, to obtain a second concentration different and preferably higher from the first concentration. Furthermore, the steps of obtaining an optical value after the addition of the (additional) second molecule is repeated to obtain a second optical value, as is obtaining the change in optical value, i.e. the change of the second optical value compared to a reference value, referred to as second change. Thus, the change of the optical value as a function of the concentration of the second molecule may be obtained. An equilibrium dissociation constant may be calculated from the concentration dependence of the first and second change.

Optionally, the method steps of providing a suspension and adding a first molecule may be repeated to obtain a second suspension. It will be understood that the method may be repeated to obtain any number of suspensions. The second molecule may be added to the second suspension to obtain a second suspension with a different, preferably higher concentrations of second molecules. The remaining method steps may then be performed on these different suspensions, in particular the second suspension, to obtain the change in the optical value as a function of the concentration of the second molecule.

The method step of obtaining a first optical value may be repeated at least once or a plurality of times until the optical value remains constant between two subsequent measurements. Preferably, the first optical value is obtained immediately, e.g. within less than 1 min, after the addition of the second molecule. Subsequently, the measurement is repeated. A kinetic constant, preferably an association or a dissociation constant, may be calculated from the time dependency of the change.

In the following, reference is made to the following proteins in **Table 1:**

| Protein name | Molar mass (kDa) | Description | Amino Acid Sequence |
|---|---|---|---|
| His$_6$-tagged TAD | 7.0 | Transactivation domain of the tumor suppressor protein p53 | MKHHHHHHPMEPLSQETFSDLWKLL-PENNVLSPLPSQAMDDLMLSPD-DIEQWFTEDPGPD |
| NCBD | 6.9 | Nuclear coactivator binding domain of CREB-binding protein (CBP) | GAMEPPRSISPSALQDLLRTLK-SPSSPQQQQQVLNILKSNPQLMAAFIKQR-TAKYVANQPGMQ |

(continued)

| Protein name | Molar mass (kDa) | Description | Amino Acid Sequence |
|---|---|---|---|
| PEX5 (1-113) | 13.3 | N-terminal domain of cytosolic peroxisomal targeting protein | MKHHHHHHPMAMRELVEAECG-GANPLMKLAGHFTQDKALRQEGLRPGPWPP-GAPASEAAS-KPLGVASEDELVAEFLQDQNAPLVSRAPQTFKMDDLLAEMQQIEQSNFRQAPQRAPGVADLA |
| PEX5 (110-230) | 15.1 | N-terminal domain of cytosolic peroxisomal targeting protein | MKHHHHHHPMADLALSENWAQEFLAAGDAV-DVTQDYNETDWSQEFISEV-TDPLSVSPARWAEEYLEQSEEKLWLGEPEGTATDRWYDEYHPEEDLQHTASDFVAK-VDDPKLANSEFLKFVRQIGEGQVSLE |
| PEX14 (16-80) | 7.6 | N-terminal domain of peroxisomal membrane-associated import protein | GAMATPGSENVLPREPLI-ATAVKFLQNSRVRQSPLATRRAFLKKKGLT-DEEIDMAFQQSGTAADEPSSLW |

It will be understood that the above recombinant proteins are shown in the following embodiments in an exemplary fashion, to illustrate the working principle of invention. The skilled person will understand that other biomolecules may be used as well.

TAD, PEX5 (1-113), and PEX5 (110-230) contain a His-tag.

In the following, the invention is described in detail with reference to the following figures, showing:

Figs. 1a-1b:     a schematic depiction and an transmission electron micrograph of a nanoparticle according to the invention.

Figs. 2a-2c:     schematically the elemental distribution of a nanoparticle according to the invention.

Fig. 3:     schematically a method of functionalizing colloidal nanoparticles according to the invention.

Fig. 4a-4b:     the molecular structure of ligands.

Fig. 5a-5b:     schematically the shape of a decahedron and a nanoparticle according to the invention.

Fig. 6:     schematically a method step of obtaining an optical value.

Fig. 7:     schematically a nanoparticle according to the invention with a protein bound to its surface.

Fig. 8:     schematically a method according to the invention.

Fig. 9a-9f:     schematically a photoreactor for producing nanoparticles according to the invention and its use.

Fig. 10a-10b:     transmission electron micrographs of nanoparticles.

Fig. 11:      stability data of nanoparticles.

Fig. 12a-12c:    refractive index sensitivity data of nanoparticles.

Fig. 13a-13d:    schematically the surface modification of nanoparticles according to the invention and related data.

Fig. 14a-14d:    data showing interaction of a His-tagged protein with a second protein.

Fig. 15:      data showing, quantitatively, the interaction between two proteins.

Fig. 16a-16b:    transmission electron micrographs of nanoparticles.

Fig. 17:      size distribution of nanoparticles.

Fig. 18a-18b:    elemental analysis by STEM-EDX of a nanoparticle.

Fig. 19a-19d:    further data related to the stability of silver nanoparticles.

Fig. 20a-20d:    further data related to the stability of silver nanoparticles.

Fig. 21a-21c:    data related to the stability of silver nanoparticles with a gold coating.

Fig. 22a-22b:    EELS data of nanoparticles according to the invention.

Fig. 23:      size distribution data for nanoparticles according to the invention.

Fig. 24:      STEM-EDX data of nanoparticles according to the invention.

Fig. 25:      data related to the stability of suspensions according to the invention in the presence of proteins.

Fig. 26:      further data related to the stability of suspensions according to the invention in the presence of proteins.

Fig. 27:      kinetic data obtained using a method according to the invention.

Fig. 28a-28f:    further data obtained by a method according to the invention.

Fig. 29:      optical data obtained from a suspension according to the invention.

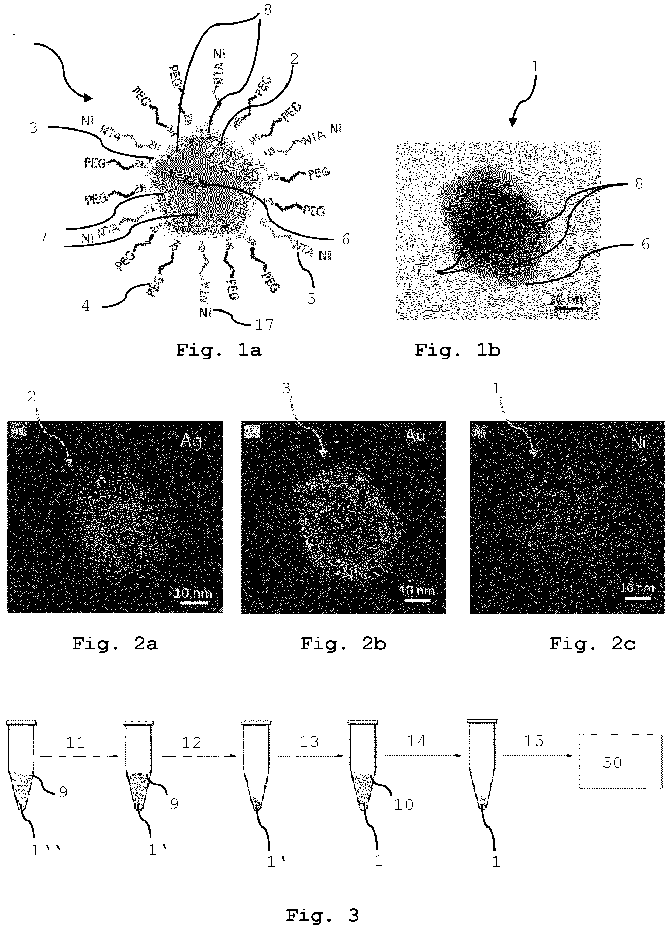

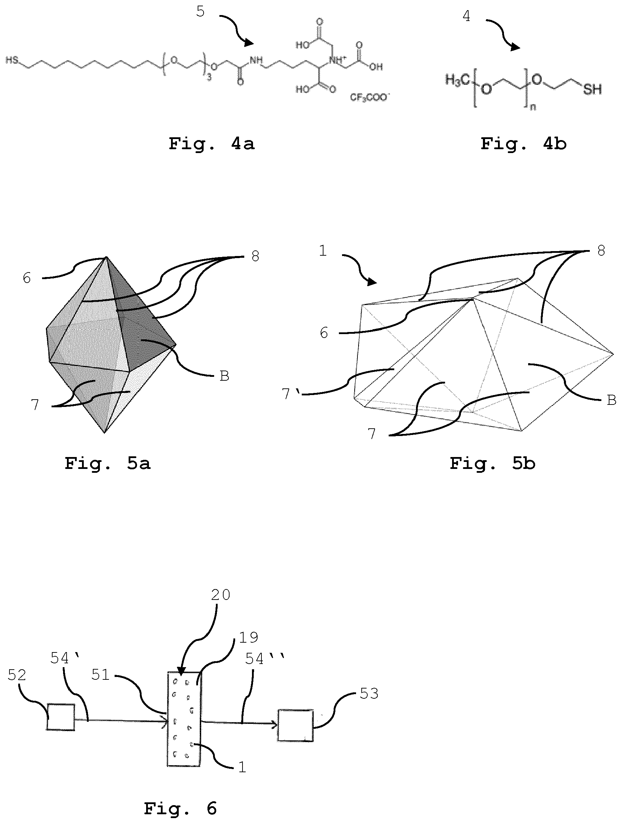

Fig. 1a shows a nanoparticle 1 according to the invention, in a schematic illustration which is not to scale. The nanoparticle 1 has a pentagonal bipyramidal shape (see Fig. 5), resulting in a decahedral shape, which is shown here along a central axis and with the pentagonal base in the image plane. The shape comprises flat facets 7 delimited by edges 8 which meet in a pyramid tip 6. It will be understood that the nanoparticle 1 has a substantially symmetrical shape relative to the image plane. The nanoparticle 1 has a core, with substantially the same shape as the nanoparticle 1 as a whole, made of silver. A gold coating 3 is arranged on the surface of the core 2. The coating 3 has a thickness of approximately 1 nm. A thiolated polyethylene glycol 4 (PEG) is attached to the gold surface of the coating 3, via the thiol end group, and acts as surface functionalization. The PEG provides colloidal stability in aqueous environments and substantially prevents non-specific binding of proteins. A second ligand 5 in the form of a thiolated nitriloacetic acid molecule (NTA, see Fig. 4) is attached to the gold coating 4, via the thiol end group. The NTA is chelated with a nickel(II) ion 17. The second ligand 5 with the nickel(II) ion 17 from a binding site, e.g. for Histidine groups which may be present, as a tag, on a protein (not shown). The nanoparticle 1 exhibits localized surface plasmon resonances (LSPR) detectable, for example, by UV/VIS spectrophotometry.

Fig. 1b shows a bright field transmission electron micrograph of the nanoparticle 1 of Fig. 1a. As understood by the skilled person, the ligands (see Fig. 1a) are not visible in the transmission electron microscopy (TEM) image. However, the shape with the edges 8, tip 6, and facets 7 is visible. The nanoparticle 1 has diameter, measured across the pentagonal base surface, of approximately 40 nm.

Figs. 2a-2c shows scanning transmission electron microscopy energy-dispersive X-ray spectroscopy (STEM-EDX) image data of the nanoparticle shown Figs. 1b.

Fig. 2a shows a silver channel, thus showing the core 2 of the nanoparticle 1.

Fig. 2b shows a gold channel, thus showing the coating 3. The stronger signal around the edges is stronger as the coating is seen, in these portions, in a side view and the signal is generated by several nanometers of material, while a middle portion has roughly coating with a thickness of approximately 1 nm leading to a somewhat weaker signal.

Fig. 2c shows a nickel channel and the signal resulting from Nickel(II) ions (see Fig. 1a) bound to the second ligands 5. To verify Ni(II)-conjugation to surface NTA ligands, the elemental composition of the surface-modified nanoparticles with scanning transmission electron microscopy (STEM)-EDX is used and shows that signal was detected on nanoparticle surfaces after incubation in $NiCl_2$ solution and redispersion in HEPES buffer. The zeta potential shifted from -32.7±0.9 mV for PEG800/40%NTA-Ni-modified nanoparticles to -28.3±1.2 mV, which is consistent with Ni pre-charging of surface NTA.

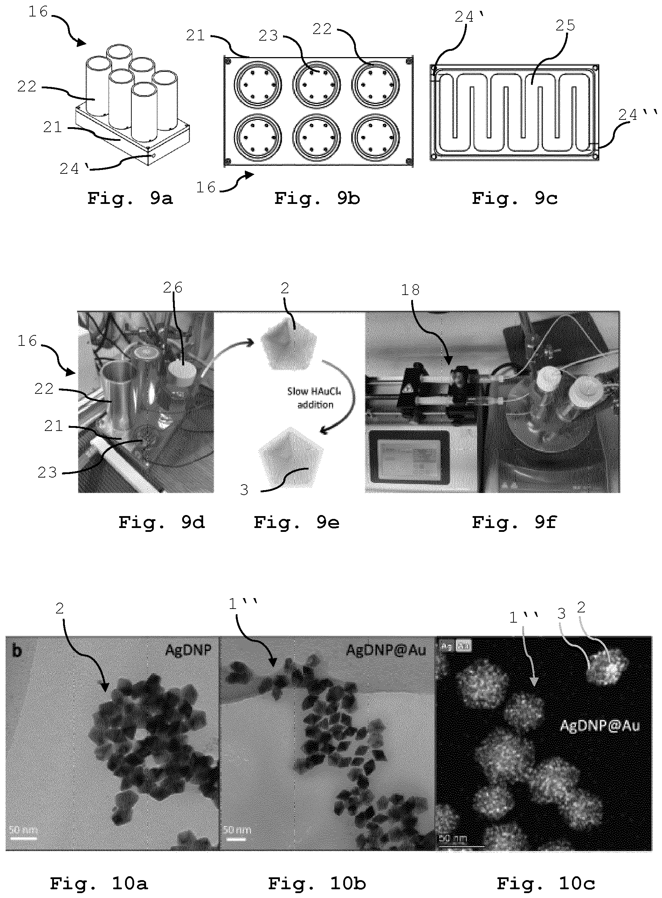

Fig. 3 shows a schematic of a workflow to produce surface-modified nanoparticles 1 as shown, for example, in Fig. 1a. Gold-coated decahedral silver nanoparticles 1'' (see Figs. 9a-9c) are provided and dispersed in an aqueous solution 9 containing poly(ethylene glycol) methyl ether thiol (in the following referred to as mPEG-SH) and 2- {2-[2-(1-mercaptoundec-11-yloxy)-ethoxy]-ethoxy}-ethoxy-nitrilotriacetic acid ($HS-(CH_2)_{11}-EG_3$-NTA, in the following referred to as "NTA", and provided in the form of a trifluoroacetate salt). The surface of the nanoparticle 1'' is prepared with mPEG-SH as a stabilizing ligand, preventing non-specific binding of proteins and colloidal stability in water-based suspensions, and NTA as a linker ligand providing a binding site for proteins, in particular His-tagged proteins.

Nanoparticles 1'' are redispersed in deionized water after centrifugation at 10870 g for 10 min before further surface modification. For a synthesis, 350 $\mu$L of nanoparticle diluted to a peak extinction of 1.16, as measured by UV-Vis spectroscopy, are placed in a 1.5 mL Eppendorf tube. The total concentration of ligands in solution is kept in excess at 375 $\mu$M, whereas the concentration of nanoparticles 1'' is around 0.1 nM. The ratio of PEG:NTA is 3:2. In an incubation step 11, the nanoparticles are incubated in the ligand solution overnight, i.e. for approximately 12 h, at room temperature and while shaking at 250 rpm.

After the incubation step, the surface of nanoparticles 1' is functionalized with the ligands PEG and NTA (see Fig. 1a, reference numerals 4 and 5) and the nanoparticles 1' are separated from the reaction mixture by centrifugation at 10870 g for 10 min. A washing step 12 is performed to remove excess ligands and repeated once. In one washing step, the PEG/NTA-modified nanoparticles 1' are redispersed in 0.1 M Tris buffer containing 0.025 wt-% Tween 20 and having a pH value of 7.6. The nanoparticles 1' are subsequently separated by centrifugation and the washing liquid is disposed, resulting in non-dispersed nanoparticles 1' after washing step 12.

Tween 20 (polysorbate 20) is known in the art and commercially available (Sigma-Aldrich, viscous liquid, cell culture tested, P2287). Tris-HCl buffer solution is commercially available (1 M, UltraPure™ Tris-HCl buffer, pH 7.5, Gibco 15567027). Tris is known in the art and an abbreviation for (tris(hydroxymethyl)aminomethane).

In a second incubation step 13, the nanoparticles 1' are incubated in solution 10, containing 50 $\mu$M $NiCl_2$, for 2.5 h at room temperature under 250 rpm shaking, resulting in nanoparticles 1 substantially corresponding to the nanoparticle shown in Fig. 1a.

In a second washing step 14, the nanoparticles 1 are washed once with 20 mM Tris buffer containing 0.005 wt-% Tween 20.

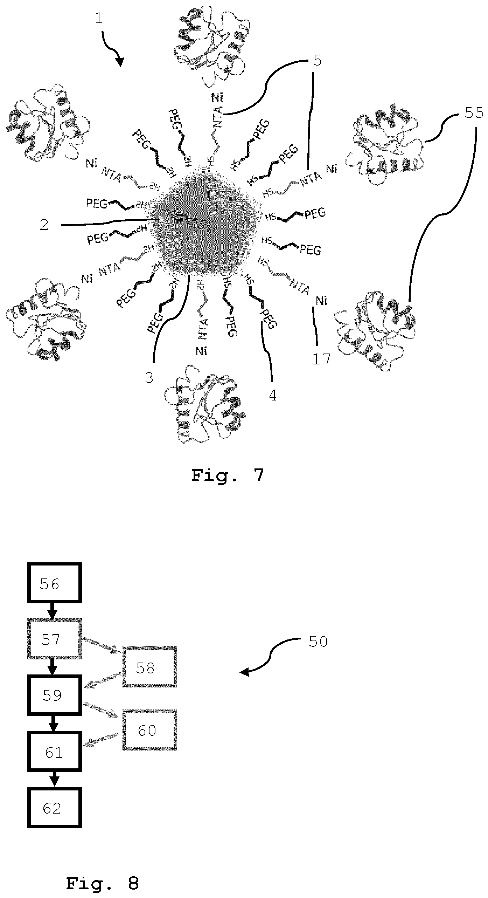

Finally, the nanoparticles 1 may be redispersed, in a redispersion step 15, in a liquid appropriate for the intended use. Here, as an example, the nanoparticles 1 are redispersed in a saline buffer known in the art having a pH of 7.5-7.9 for subsequent use in a method 50 of sensing protein-protein interactions via a spectrophotometer (see Figs. 6 and 8).

mPEG-SH is commercially available from Sigma-Aldrich (Prod. No. 729108 / MW = 0.8 kDa; Prod. No. 729140 / MW= 2 kDa). NTA is commercially available from Prochimia Surfaces (Gdansk, Poland, Prod. No. TH 007).

Fig. 4a shows the molecular structure of NTA trifluoroacetate. NTA may be used as ligand 5 providing a binding site for a protein (see Fig. 1a and Fig. 7).

Fig. 4b shows the molecular structure of a thiolated PEG which may be used as a ligand (see Fig. 3). Here, n is between 16 and 21.

Fig. 5a shows schematically an ideal pentagonal bipyramid. A pentagonal base surface B is shown for ease of understanding. Side facets 7 extend from the base surface B toward tip 6 and separated by edges 8. The shape of the pentagonal bipyramid is substantially symmetrical on both sides of the pentagonal base surface B.

Fig. 5b shows schematically the shape of a nanoparticle 1 according to the invention. The shape substantially corresponds to a pentagonal bipyramid as shown in Fig. 5a. However, when the nanoparticle 1 comprises a silver core and a gold coating (see Fig. 1), due to the crystal symmetry a sixth side facet 7' may be present which is smaller than facets 7. The sixth facet 7' is placed and oriented where an edge 8 would be in a perfect pentagonal bipyramid and has the appearance of a cut-off edge 8. Therefore, the nanoparticle 1 may not have a perfect five-fold rotation symmetry. The skilled person will understand that any reference herein to a decahedral shape (or pentagonal bipyramid) may include the shape shown here.

Fig. 6 shows schematically the measurement of an optical value of a suspension 20. The suspension contains nanoparticles 1 as shown in Fig. 1a and a saline buffer. The suspension 19 is placed in a 1.5-mL semi-micro polystyrene cuvettes 51 pre-coated with bovine serum albumin (BVA). A light source 52 is used to illuminate the cuvette 51 with selective wavelengths and a photodetector 53 is used to measure the light transmitted through the cuvette 51. For example, a commercially available Jasco V-760 UV-Vis spectrophotometer may be used for collecting the extinction spectra of suspensions 20 of nanoparticles 1. It will be understood that the light path 54', 54'' is shown in a schematic way.

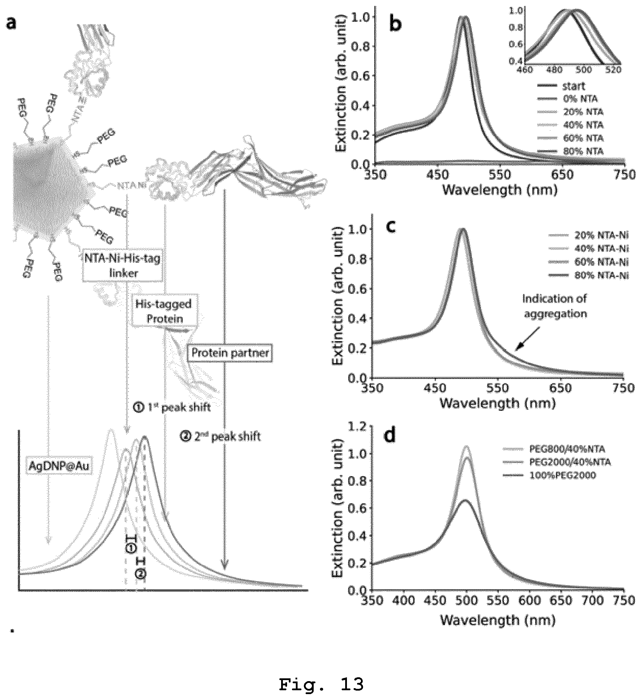

Fig. 7 shows a nanoparticle 1, as shown in Fig. 1a. Here, a protein 55 is attached to the binding site formed by the NTA ligands 5 and the chelated Nickel(II) ions 17. In principle, any protein with a poly-histidine tag may bind to the binding site of the shown nanoparticle 1. A molecule bound to the surface of nanoparticle 1, such as proteins 55, may shift a LSPR peak (see Figs. 10-29). Further shifts may occur when another molecule, for example a second protein (not shown, see Fig. 13) interacts with the proteins 55.

Suitable proteins are, for example, $His_6$-tagged TAD and/or His-tagged versions of NCBD, PEX5(1-113), PEX5(110-230), or PEX14(16-80).

Fig. 8 shows schematically the steps of a method 50 according to the invention for measuring interaction between to molecules. In an exemplary fashion, the method is described with respect to a first and a second protein. In a first step 56, a suspension of nanoparticles 1 in a suitable buffer is provided. If the nanoparticles 1 contain a binding site for a protein, for example, an optional step 57 of adding a first protein may be performed. This first protein may then bind to the binding site so as to subsequently study the interaction between this first protein and another molecule, e.g. a second molecule. Alternatively, the suspension may be provided with a surface bound molecule whose interaction with a second molecule is to be studied. Further optionally, a waiting step 58 may be performed after addition of the first protein to allow for substantially all binding sites to be bound to a first protein. Subsequently, a second protein is added 59, and optionally a second waiting step 60 is performed to let an equilibrium be reached between the first and the second protein. Subsequently, an optical value is measured, for example an extinction spectrum measured with a UV/VIS spectrophotometer and a peak position representing a localized surface plasmon resonance (LSPR) is determined. The peak position is then compared to a reference value in a final step 62. The reference value may be known, based on the suspension and first molecule used, but the skilled person will understand that reference value may be obtained by measuring the optical value before step 59. Furthermore, it will be understood that the method steps may be repeated, e.g. on several identical suspensions and by using different amounts of second proteins. As will be shown below, the obtained spectral peak changes as a function of the concentration may be used to calculate equilibrium constants for the interaction between the first and the second molecule. Additionally or alternatively, the step 61 may be started substantially immediately after the addition of the second protein and performed repeatedly. The change of the optical value as a function of time may be used to calculate kinetic constants, as will also be shown in more detail below.

It will be understood that after each step, if required for a given application, a spectrum (UV/VIS) may be measured and the method continued once the spectrum is stable, i.e. does no longer change.

Fig. 9a-9c show, schematically, the synthesis of gold-coated silver decahedral nanoparticles 1''.

Fig. 9a shows schematically a photoreactor 16. The photoreactor 16 comprises an aluminum block 21 with an integrated cooling system (not visible, see Fig. 9c), LEDs 23 (not visible, see Fig. 9b), and vial holders 22. Six vial holders 22 are positioned on top of the LEDs. The entire photoreactor may be placed on a shaker so that mixing may occur without the use of stir bars.

Fig. 9b shows the photoreactor 16 of Fig. 9a in a top view. For clarity, identical features are not described again. Six LEDs 23 (here, for example, 455 nm ILH-ON09-DEBL-SC211-WIR200 from Intelligent LED Solutions) are screwed onto the block along with thermal paste on the LED backs. The vial holders 22 are positioned to maintain a spacing of 10 mm between the LED 23 and the bottom of a scintillation vial (see Fig. 9d) which may be placed in a vial holder 22.

Fig. 9c shows a cross-section of the aluminum block 21 of the photoreactor of Figs. 9a and 9b. The aluminum block 21 contains a water inlet 24' and outlet 24'' with a meandering path 25 and is connected to a 5 L water basin via a pump (not shown). The cooling provided by water flowing through the meandering path 25 ensures that solution temperatures does not increase by more than 0.1 degrees Celsius during illumination, and additionally cools the LEDs, so that output light intensity is stable (140 mW at LED, 25 mW at the top of the scintillation vial).

Fig. 9d shows a photographic depiction of a use of the photoreactor 16 of Figs. 9a-9b. Here, only two vial holders 22 are present, exposing an LED 23. A scintillation vial 26 is shown outside of a vial holder 22 for illustration. To synthesize nanoparticles, in a 20 mL glass scintillation vial, 14 mL deionized water is added, followed by the following compounds in the order listed: 0.52 mL 50 mM sodium citrate, 0.023 mL 2 mg/mL polyvinylpyrrolidone (MW = 40 kDa), 0.025 mL 5 mM L-Arginine, 0.4 mL 5 mM $AgNO_3$, 0.2 mL freshly prepared 0.1 M $NaBH_4$. After "aging" at room temperature for 50 min in the dark, a bright yellow solution forms. The vial is then placed in the photoreactor and shaken at 250 rpm for 10 minutes in the dark. Directly after, the 455 nm LED is turned on (140 mW at LED), and 0.3 mL 30% $H_2O_2$ is added while the vial is still shaking. The shaking is maintained for 30 min and then turned off. The vial is exposed to LED light for 14.5 h in total to yield the Ag decahedral nanoparticles.

As shown in Fig. 9e, a gold coating 3 of silver decahedral nanoparticles 2 is then performed. 3 mL of aqueous 0.0128 mM $HAuCl_4$ solution is added to 3 mL of suspension, the reaction product according to the protocol described in the context of Fig. 9d without additional steps, containing of silver nanoparticles 2 at an addition rate of 0.25 mL/h over 12 h.

The reaction may be performed under stirring at 200 rpm at room temperature and in the dark. The molar ratio of $HAuCl_4$ in the final suspension is 1/10 of the $AgNO_3$ used for preparing the silver decahedral nanoparticle suspension. The obtained nanoparticles 1'' may be separated from their growth solution by centrifugation at 2500 g for 30 min, and then redispersed in deionized water.

Fig. 9f shows, in a photographic depiction, a syringe pump 18 used to add the 3 mL of aqueous 0.0128 mM $HAuCl_4$ solution at an addition rate of 0.25 mL/h over 12 h.

The synthesis protocols for obtaining silver nanoparticles with a decahedral shape and for their subsequent gold plating are known in the art and published by Murshid and Kitaev (DOI: 10.1002/ppsc.201300225 and DOI: 10.1039/C3CC46075D, respectively) .

In the following, data related to the characterization of nanoparticles according to the invention as well as the detection of molecular interaction is shown. For ease of understanding, decahedral silver nanoparticles, i.e. the cores (see Fig. 1a) of nanoparticles according to the invention, will be referred to as "AgDNP". As an exemplary embodiment of nanoparticles according to the invention, decahedral silver nanoparticles with a gold coating and surface functionalization are used in the following and may be referred to as "AgDNP@Au". The data shown in the following was thus obtained using nanoparticles as shown in Fig. 1a. It will be understood that surface functionalizations as described herein may be present on the nanoparticle surface. The following abbreviations will be used, which are known to the person skilled in the art: TEM (transmission electron microscopy), STEM (scanning transmission electron microscopy), EDX (energy-dispersive X-ray spectroscopy), EELS (electron energy loss spectroscopy), DLS (dynamic light scattering), RI (refractive index).

Figs. 10a-10c show TEM images of AgDNP (Fig. 10a) and Au-coated AgDNP (Fig. 10b), and STEM-EDX image of AgDNP@Au with Ag (blue) and Au (yellow) channels overlaid (Fig. 10c).

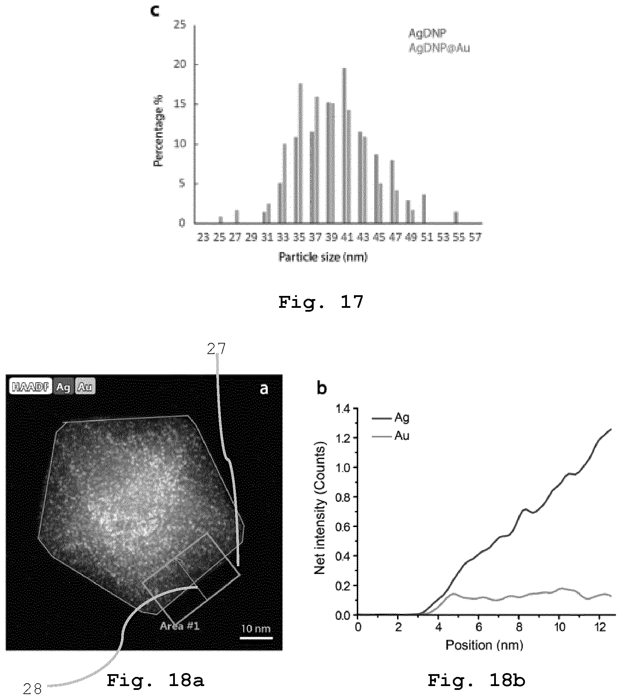

TEM showed that the average heights of the pentagon plane of the AgDNP and AgDNP@Au were 38.4 nm and 41.0 nm, respectively (Fig. 10a-10c), for size distributions see Fig. 17). This slight difference was also reflected in DLS, which showed hydrodynamic diameters of ~38.7 nm and ~42.5 nm for the particles before and after Au coating, respectively. The thickness of the Au layer was estimated to be roughly 1 nm, or 3-4 atomic layers, by STEM and EDX analysis (Fig. 18a-18b).

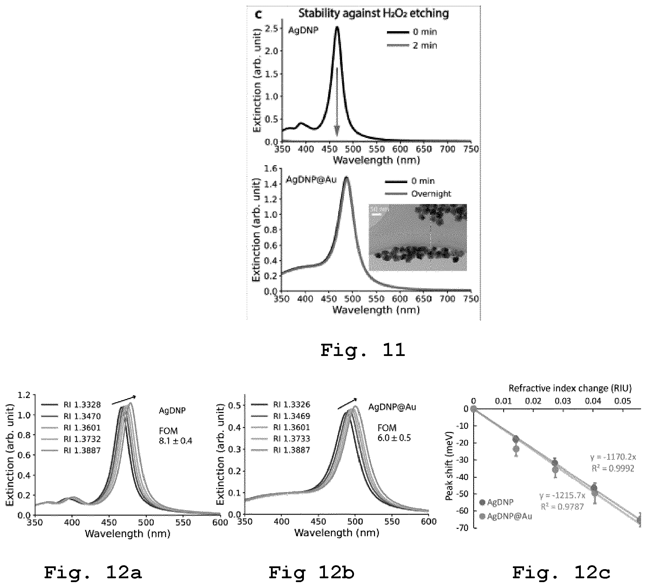

Fig. 11 shows a stability test of AgDNP (top) and AgDNP@Au (bottom) against 1.0 M $H_2O_2$ etching, showing a complete gold coating around the silver core. The TEM image inset shows AgDNP@Au after $H_2O_2$ etching overnight.

Non-spherical Ag nanoparticles are known to be chemically unstable as release of Ag atoms from high-energy facets occurs even under mild conditions. In an oxidative environment, nanoparticles can be etched away within minutes. Fig. 11 shows the stability of AgDNP and AgDNP@Au in 1.0 M (3%) $H_2O_2$ solution before and after Au coating. The extinction spectrum of AgDNP@Au remained nearly unchanged after overnight exposure, with the large majority of nanoparticles remaining intact, as also seen in TEM (inset of Fig. 11, bottom). In contrast, the LSPR band of uncoated AgDNP vanished nearly instantly. These results suggest that the ultra-thin Au coating was not only essentially complete around the AgDNP cores, but also sufficiently thick to protect the cores from oxidative damage.

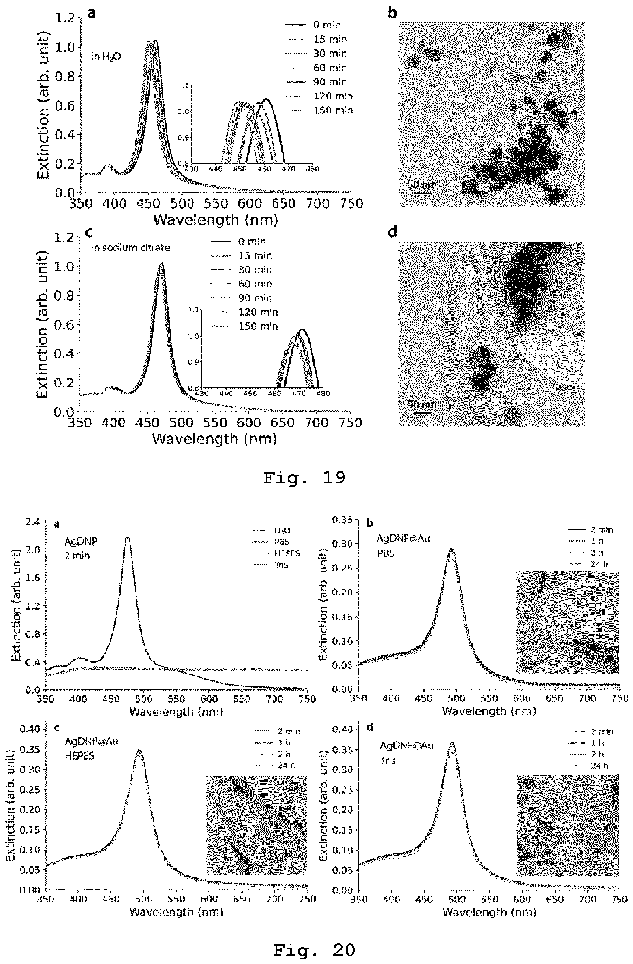

Figs. 12a-12c show LSPR spectra in different RI solutions, from which the RI sensitivity of different nanoparticles may be calculated. Fig. 12a shows AgDNP and Fig. 12b shows AgDNP@Au in glycerol-water solutions, with black arrows indicating the increasing RI. RI values were confirmed with a refractometer. In measurements of AgDNP, 2 mM sodium citrate was included in the solution to improve nanoparticle stability (see Fig. 19). The spectrum amplitude was corrected for dilution. Fig. 12c shows a linear fitting of spectral peak shifts vs. RI change which may be used to derive RI sensitivities (RIU/meV).

Optically, the extinction spectra of the Ag decahedral nanoparticles showed a sharp peak around 472 nm with full-width at half-maximum (FWHM) of 140 meV attributed to the longitudinal dipole localized surface plasmon resonance (LSPR) mode (Fig. 11 and Figs. 12a-12c). The AgDNP@Au nanoparticles present a red-shifted LSPR band around 492 nm with a broadened FWHM of 228 meV, due to higher plasmonic damping of Au. It is also observed that the transverse dipolar LSPR mode of AgDNP around 401 nm is significantly diminished after Au coating (Fig. 11 and Figs. 12a-12c). A thin Au layer improves chemical and colloidal stability of the nanoparticles without considerably hampering the optical-sensing properties of the Ag core.

The sensing ability of LSPR optical sensors is typically quantified with RI sensitivity and the so-called figure-of-merit (FOM), which is the ratio of the RI sensitivity and the LSPR spectral linewidth. A large FOM is normally desired because a smaller perturbation, such as the presence of a protein, results in a more evident spectral shift. The RI sensitivity and FOM of AgDNP and AgDNP@Au are determined by measuring their spectral shifts in varying glycerol-water mixtures, with the refractive index verified with a refractometer.

Together with reported literature values, the RI-sensitivity and FOM values are summarized in **Table 2**:

| Nanoparticles | size (nm) | RI sensitivity (meV/RIU) | FOM |
|---|---|---|---|
| Au rod | 73x41 | 640 | 2.1 |
| Au cube | 77 | 580 | 1.5 |

(continued)

| Nanoparticles | size (nm) | RI sensitivity (meV/RIU) | FOM |
|---|---|---|---|
| Au sphere | 15 | 200 | 0.6 |
| Au bipyramid | 27x19 | 450 | 1.7 |
| Ag sphere | 40 | 1100 | 2.6 |
| Ag triangular prism | 88x24 | 1200 | 4.6 |
| Ag cube | 84 | 1400 | 1.2 |
| Ag cube* | 84 | 1000 | 4.6 |
| Ag@SiO$_2$ | 106 | 750 | 1.7 |
| Au@Ag rod | 49x25 | 900 | 3.1 |
| **AgDNP** | **38** | **1200** | **8.1** |
| **AgDNP@Au** | **41** | **1200** | **6.0** |

The AgDNP disclosed herein possess a longitudinal SPR mode at 472 nm, along with a high RI sensitivity at 1170 $\pm$ 51 meV/RIU. This value approaches some of the highest sensitivities for colloidal nanoparticles known in the art, such as 88 $\times$ 24 nm Ag triangular prisms and 84 nm Ag nanocubes (see Table 1 above), despite a smaller physical size. However, the AgDNP reached a FOM of 8.1 $\pm$ 0.4 which is nearly seven times the FOM of 84 nm Ag nanocubes, in large part due to the difference in spectral linewidth.

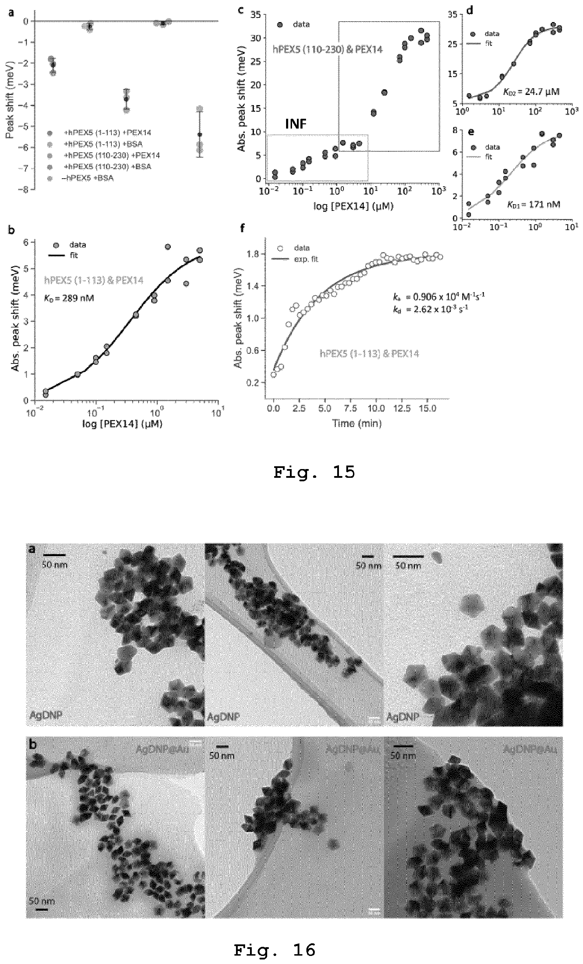

Unexpectedly, it was found that gold overcoating does not significantly reduce the RI sensitivity of AgDNP@Au (1216 $\pm$ 111 meV/RIU) compared to AgDNP (Fig. 12c). This is a rather surprising finding as for metallic nanoparticles with similar shape, size and LSPR wavelength, the smaller real part of the dielectric function and the stronger plasmonic damping of Au compared to Ag should lead to a larger RI sensitivity of Ag nanoparticles. This might be attributed to the high RI sensitivity of AgDNP@Au to the ultrathin surface Au layer (~1 nm), and a possible sharpening of Ag tips during the coating process (see TEM images in Fig. 10b and Fig. 16).

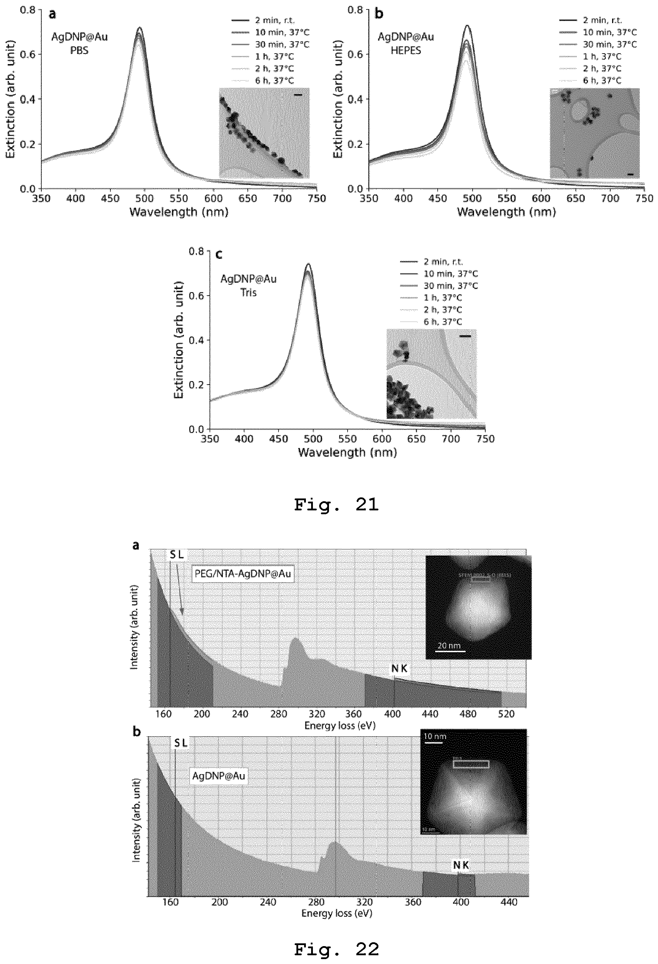

Furthermore, the stability of AgDNP and AgDNP@Au in commonly used biological buffers including phosphate-buffered saline (PBS), HEPES- and Tris-buffered saline buffers are examined. It was found that, after Au coating, the nanoparticle colloidal stability was substantially improved up to 24 h in all tested buffers (see Fig. 20). An adequate stability was observed at the physiological temperature of 37°C up to 6 h as well (Fig. 21).

Therefore, AgDNP@Au show a high sensing capability combined with enhanced chemical and colloidal stability and are therefore particularly suitable for interfacing with, and sensing, proteins at their surface.

Figs. 13a-13d show schematically the surface modification of AgDNP@Au for protein-protein interaction sensing and related data.

The gold surface of AgDNP@Au not only improves stability of the nanoparticles in biological buffers but also permits covalent Au-thiol chemistry to introduce an array of surface ligands.

Here, a strategy for adapting the surfaces of the AgDNP@Au for nano-bio interactions involves a combination of "stabilizing ligands" and "linker ligands" to achieve high colloidal stability, modular design, and specific protein immobilization.

Thiolated polyethylene glycol (PEG-SH) and its derivatives are suitable surface ligands for stabilizing noble metal nanoparticles in buffers and conferring biocompatibility. Attaching PEG molecules with high molecular weights (MW) may improve the colloidal stability of nanoparticles and reduces non-specific interactions between proteins and particle surfaces through steric hindrance. However, excessively large ligands might also reduce the nanoparticle's ability to sense biomolecules, as longer surface ligands may restrict access of analyte biomolecules to the LSPR hotspots at the nanoparticle surface. Methoxy PEG thiol (mPEG-SH) with low MW (~0.8 kDa, "PEG800") is therefore particularly suitable to function as the "stabilizing ligand".

These "stabilizing ligands" may be used in combination with a "linker ligand" to specifically tether proteins to the nanoparticle surface. Suitable linkers include biotin-streptavidin linkage, protein-A/G-IgG linkage,46 and Ni(II)-Nitrilotriacetic acid (NTA)-poly(6)-Histidine ("NTA-Ni-His-tag") linkage. As shown herein, Ni(II)NTA functional groups may be immobilized onto AgDNP@Au surfaces in order to bind proteins close to the nanoparticle surface for maximal spatial overlap with the LSPR mode (Fig. 13a).

Specifically, a thiolated alkane-PEG-NTA chelated with Ni(II) ("NTA-Ni") may be used to bind to the His-tag of recombinantly produced proteins (see Figs. 4a-4b for chemical structures of PEG800 and NTA). With a molecular weight of ~1.5 kDa including a His$_6$-tag, this linkage is smaller than other common linkers such as biotin-streptavidin (~53 kDa), allowing to bring the protein analyte much closer to the nanoparticle surface. Moreover, NTA-Ni-His-tag linkage may allow for straightforward modularity with many recombinant proteins.

Fig. 13a shows a schematic of protein-protein interaction sensing via a NTA-Ni-His-tag linker and the stepwise change in LSPR band from refractive-index sensing. The "1st peak shift" and "2nd peak shift" may be understood as the spectral shift, as measured in UV/VIS, after attachment of the His-tagged protein and the protein partner, respectively.

Figs. 13b shows the effect of NTA:PEG800 ratio on LSPR peak after surface modification. The total ligand to nanoparticle ratio and amount are kept the same. Nanoparticles are redispersed in $H_2O$ for measurements. The spectra are normalized except for 0% NTA (100% PEG800)-modified particles, which could not be redispersed. The insert shows a zoom-in of the spectral peak.

In general, here and in the following, nanoparticles were redispersed in different saline buffers in BSA pre-coated cuvettes for protein sensing experiments. The BSA pre-coating was performed using a 2.5% (w/v) BSA in PBS buffer, shaken thoroughly to touch all surfaces of the cuvettes for 20 min before removal. The coated surfaces were then washed with Tris-buffered saline and deionized $H_2O$, and dried using compressed air. Desired amounts of protein stock solution were added to the nanoparticle suspensions. The extinction spectrum (350 - 750 nm, 0.2 nm intervals) was monitored until no further peak shift was observable. 250 rpm shaking was applied between measurements to facilitate mixing.

Fig. 13d shows extinction spectra of nanoparticles after PEG800/(20-80%)NTA-Ni surface modification. Particles were redispersed in 20 mM HEPES, 150 mM NaCl, 0.025 wt% Tween 20, pH 7.5 buffer.

Fig. 13e shows the effect of mPEG-SH length used in PEG/NTA surface modification on LSPR peak. For comparison, the spectrum of nanoparticles modified with 100% PEG2000 is shown.

As shown in Fig. 13b, surface modification with PEG800 and with 20% NTA, nanoparticle colloidal stability is enhanced, and a +2.0 nm (-10.4 meV) redshift along with a peak width broadening by 23%, is observed. Higher NTA ratios of 40-80% yields redshifts of +6.6-8.0 nm (-34.0 to -41.1 meV), which reflects surface ligand attachment providing adequate specific linkage sites. In addition, compared to 20% NTA, peaks experience less broadening (12.5-15.3%), suggesting reduced plasmonic damping with increasing NTA and decreasing PEG800. EELS confirms the appearance of sulfur and an intensified nitrogen signal on the modified particle surface after ligand incorporation (see Fig. 22).

PEG800/NTA-Ni surface-modified AgDNP@Au are investigated after incubation in excess $NiCl_2$ (50 $\mu M$ NiCl2 to ~0.1 nM nanoparticles).

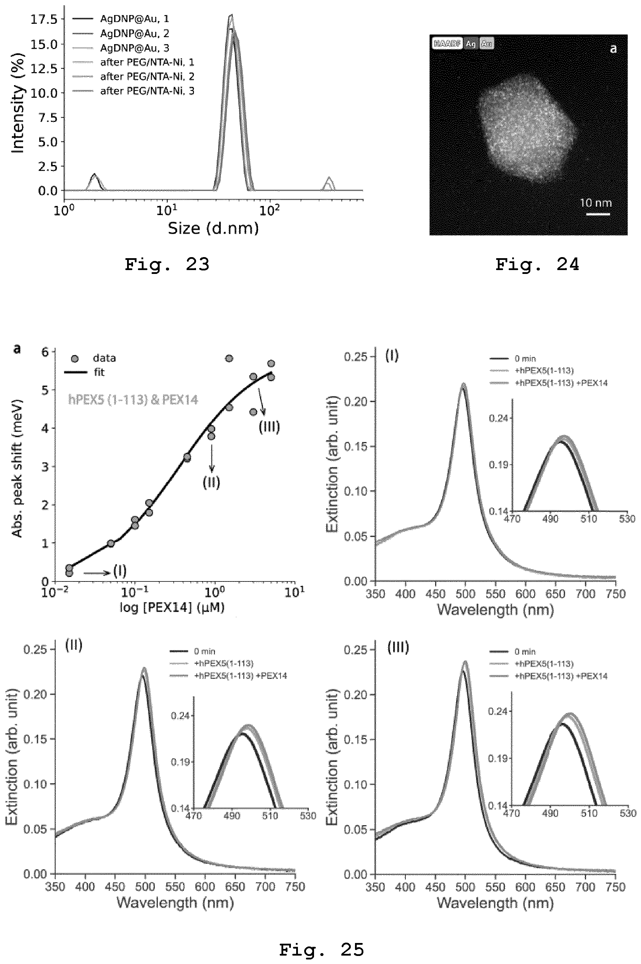

In Fig. 13c, nanoparticles modified with 20-60% NTA-Ni (i.e. 80-40% PEG800 correspondingly) were redispersible in HEPES saline while maintaining their spectra. DLS does not indicate any aggregate formation (Fig. 23) after PEG800/40%NTA-Ni modification, and also reveals a nanoparticle hydrodynamic diameter increase of ~3.1 nm. With 80% NTA-Ni, a shoulder appears in the spectrum at ~550 nm upon nanoparticle redispersion in HEPES saline, indicating the formation of nanoparticle aggregates. Taken together, the spectra of Figs. 13b and 13c and DLS measurements (see Fig. 23) indicate that 40-60% NTA, with corresponding 40-80% PEG800, provided adequate linker sites, while also maintaining colloidal stability of AgDNP@Au in saline buffers after Ni(II)-NTA conjugation. In certain embodiments, 40% NTA-Ni may thus be selected as a preferred ligand combination as particles are not susceptible to aggregation or LSPR peak broadening, even when larger mPEG-SH (MW ~2 kDa, "PEG2000") was used as the stabilizing ligand (Fig. 13d).

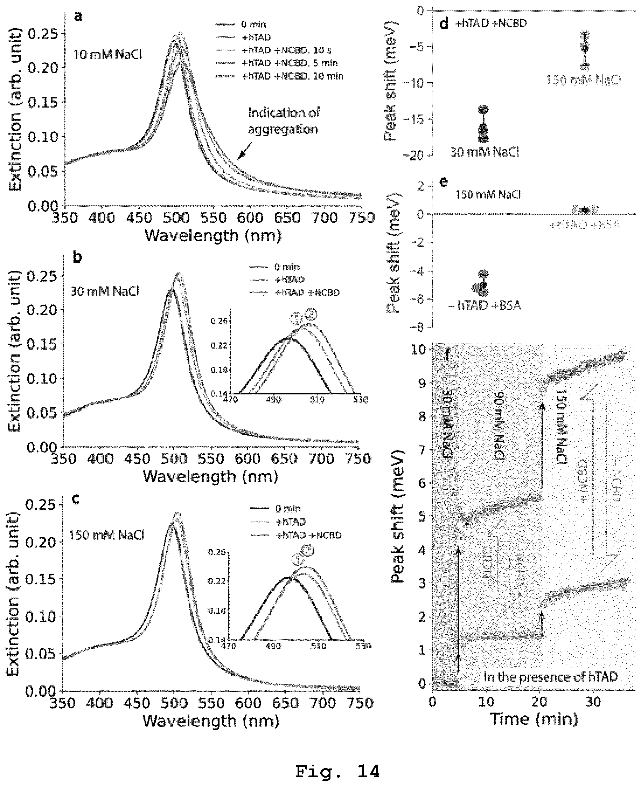

Fig. 14 shows measurement data related to His-Tagged TAD ("hTAD", 0.16 $\mu M$) and NCBD (0.27 $\mu M$) interaction in 10 mM Tris buffer, 0.025wt% Tween 20, pH 7.5, and further containing 10 mM NaCl (Fig. 14a), 30 mM NaCl (Fig. 14b), and 150 mM NaCl (Fig. 14c). Details on the proteins used here are shown in Table 1. The nanoparticle concentration is kept at an estimated ~0.025 nM. The insets in Figs. 14b and 14c show zoom-ins of spectral peaks. Spectral intensities are corrected for the dilution effect from protein addition. ① and ② indicate the 1st and 2nd peak shifts, respectively.

Fig. 14d shows the 2nd peak shifts after addition of NCBD to hTAD-immobilized nanoparticles at 25 min (near equilibrium) in Tris buffer containing 30 mM and 150 mM NaCl.

Fig. 14e shows the peak shifts after addition of 1.6 $\mu M$ BSA to hTAD-immobilized and bare nanoparticles at 25 min (near equilibrium) in Tris buffer containing 150 mM NaCl. Black dots and error bars in Figs. 14d and Figs. 14e represent the mean values and standard deviations of triplicate experiments, respectively.

Fig. 14f shows the stepwise LSPR-peak blue shifts after changing NaCl concentration from 30 mM to 90 mM, then to 150 mM. The salt concentration effect was measured for nanoparticles attached with hTAD alone for comparison ("-NCBD").

In Figure 14a-c, the sensing of the interaction between His-tagged TAD ("hTAD", 7.0 kDa) and NCBD (6.9 kDa) is evaluated by recording the spectral changes of the nanoparticles upon two-step protein addition in Tris-based buffers at different NaCl concentrations. At 10 mM NaCl (Fig. 14a), a redshift of +6.2 nm (-30.4 meV) is observed after 10 s manual mixing, but several minutes afterwards, the peak broadens with a red shoulder and reduced intensity, an indication of nanoparticle aggregation. The binding affinity of TAD and NCBD is highest at 10 mM NaCl ($K_D$ = 0.104 $\mu M$), among the three NaCl concentrations shown here. Under these conditions, multiple contacts could occur between a single soluble NCBD protein and several TAD-nanoparticle complexes, leading to the formation of nanoparticle oligomers.

When increasing the NaCl concentration to 30 mM and 150 mM, the second peak redshift decreases to +3.2 nm (-15.5 meV) and +1.0 nm (-4.9 meV) (Fig. 14b-d). These findings match the reported trend of reduced binding affinities at higher salt concentrations, as a larger redshift is expected for lower $K_D$ as more NCBD binds to nanoparticle-immobilized hTAD at the same solution NCBD concentration ($K_D$ = 1.6 $\mu$M at 30 mM NaCl to 29.9 $\mu$M at 150 mM NaCl). The spectral lineshapes remained narrow throughout the measurements, indicating stable colloidal dispersions.

As shown by the spectrum of a single nanoparticle sample containing hTAD and NCBD undergoing addition of NaCl (Fig. 14f), the spectral shifts arose from the protein-protein interactions themselves. Rapid, stepwise blue shifts in the LSPR peak position are observed when increasing the NaCl concentration first from 30 mM to 90 mM, then to 150 mM, indicative of enhanced dissociation at higher salt concentrations. Smaller blue shifts are observed for nanoparticles immobilized with hTAD alone, in the absence of NCBD, as shown in Fig. 14f. Altogether, these results indicate that the specific protein interaction between hTAD and NCBD is responsible for the observed LSPR spectral changes described in Figs. 14a-14f.

Fig. 14e shows data further verifying the specificity of the protein-protein interaction detected. In a control experiment, the protein binding partner (NCBD) was replaced by a protein not expected to bind to TAD, bovine serum albumin (BSA, 66.5 kDa). As shown in Figure 14e, BSA addition to hTAD-immobilized nanoparticles led to essentially no spectral shift, confirming both the lack of binding between BSA and TAD, in contrast to NCBD addition to TAD (Fig. 14d). Non-specific interaction sites on the nanoparticle surface may be further minimized, permitting the detection of specific protein-protein interactions, with sufficient hTAD loading.

Fig. 15 shows data related to the quantification of PEX5-PEX14 interactions. Details on the proteins PEX5 and PEX14 are shown Table 1.

Fig. 15a shows LSPR peak shifts caused by addition of 0.15 $\mu$M PEX14 (blue and green dots) or 0.15 $\mu$M BSA (gold and orange dots) to nanoparticles decorated with hPEX5 (1-113) or hPEX5 (110-230) at 0.015 $\mu$M. The peak shifts caused by 0.15 $\mu$M BSA addition to nanoparticles without any PEX5 are shown for comparison (violet dots). Black dots and error bars represent the mean values and standard deviations of triplicate experiments, respectively.

Fig. 15b shows the binding curve of PEX5 (1-113) and PEX14 (16-80) pair.

Fig. 15c shows the binding curve of PEX5 (110-230) and PEX14 (16-80) pair.

Fig. 15d shows a zoomed-in view of the high-concentration parts of the full binding curve in Fig. 15c.

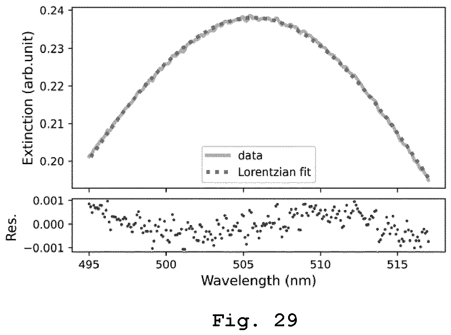

Fig. 15e shows a zoomed-in view of low-concentration parts of the full binding curve in Fig. 15c. Hill-Waud equation is used for the fittings in Figs. 15b-15e (see Fig. 29).

Fig. 15f shows a typical kinetic measurement of the PEX5 (1-113) and PEX14 (16-80) interaction without pre-mixing at a PEX14:PEX5 ratio of 10:1. For each measurement, 0.015 $\mu$M hPEX5 construct is added to ~0.025 nM nanoparticles in a 20 mM sodium phosphate buffer containing 100 mM NaCl and 0.025wt% Tween 20 at pH 7.9. The solid line is an exponential fit through the data. In similar experiments, the binding specificity between both PEX5 constructs and PEX14 can be demonstrated using BSA (Fig. 15a). Similar to hTAD, the attachment of either His-tagged PEX5 construct ("hPEX5"), hPEX5 (1-113) or hPEX5 (110-230), prevents non-specific binding of BSA with the nanoparticle surface. However, significant peak redshifts are observed in the presence of the PEX5 binding partner PEX14 (16-80). These experiments are a further demonstration of the modularity of this sensing platform, as the same nanoparticles could be used for either set of protein binding partners for measuring specific protein-protein interactions.

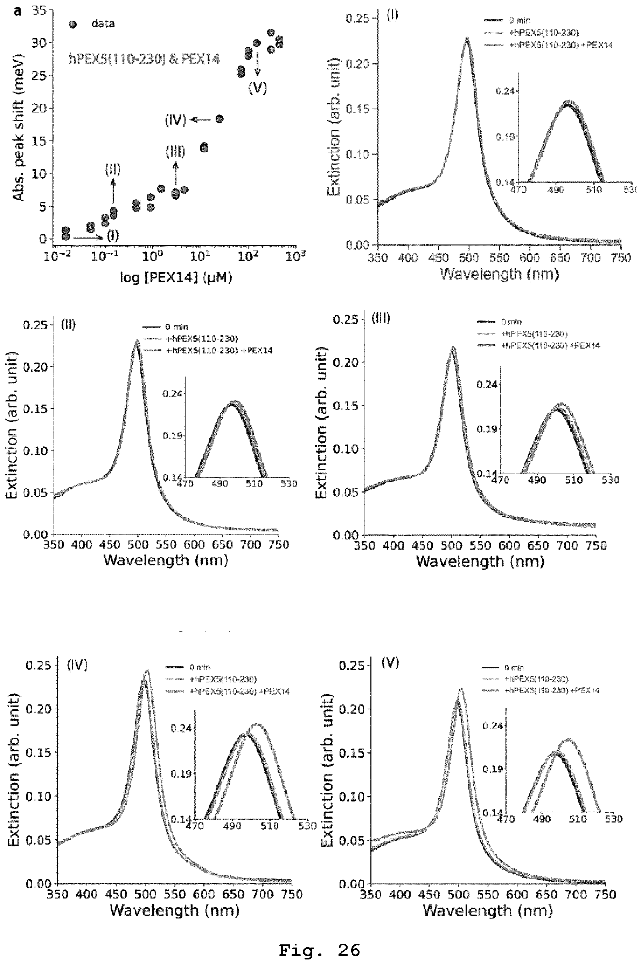

Beyond qualitative protein-protein sensing, the nanoparticles of the invention may be used for quantifying binding affinities and kinetics. Figure 15b-15e show the magnitude of shifts in the LSPR peak position caused by addition of varying concentrations of PEX14 (16-80) to either of the two His-tagged PEX5 constructs tethered to the nanoparticles. The nanoparticles maintained colloidal stability throughout the entire binding curves, spanning a dynamic range of up to 5 orders of magnitude in PEX14 concentration (see Figs. 25 and 26). The equilibrium dissociation constant ($K_D$) may be derived from each of the binding curves. $K_D$ of PEX5 (1-113) and PEX14 was determined to be 289 nM.

The kinetic change in the LSPR peak for PEX5 (1-113) and PEX14 binding is shown in Figure 15f. The average observable rate $k_{obs}$ is 0.00398$\pm$0.00220 s-1, as determined from the average of three separate measurements and their fits to exponential functions (see also Fig. 29). The association and dissociation rate constants $k_a$ and $k_d$ are derived from $k_{obs}$ and $K_D$ to be 0.906 $\times$ 10$^4$ M$^{-1}$s$^{-1}$ and 2.62 $\times$ 10$^{-3}$ s$^{-1}$, respectively.

PEX5 (1-113) contains a single motif W0, whereas PEX5 (110-230) carries four diaromatic peptide motifs W1-W4 that can interact with PEX14 (16-80). Motifs W1-W3 bind strongly to PEX14, showing small $K_D$ values in a narrow range of 139-344 nM, as measured by ITC. The farthest motif W4 interacts much more weakly with PEX14 and exhibits a $K_D$ of 6.3 $\mu$M. These disparate binding constants are reflected in the two inflection points of the binding curve of PEX5 (110-230) and PEX14 in Figure 15c.

Each inflection point may be analyzed separately using the Hill-Waud equation (see Fig. 29) to give $K_{D1}$ = 171 nM and $K_{D2}$ = 24.7 $\mu$M (see Figs. 15d and 15e). $K_{D1}$ may be attributed to the strong binding between motifs W1-W3 on PEX5(110-230) and PEX14. Different Hill's coefficients were observed for the 1st and 2nd inflection points ($n_1$=0.838 < 1, $n_2$ = 1.19 > 1) of PEX5 (110-230) and PEX14.

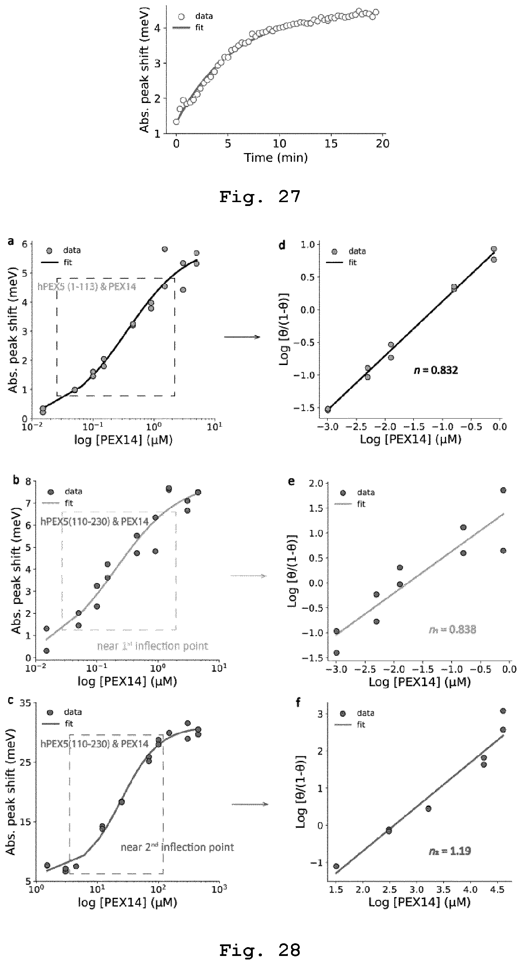

As with the PEX5 (1-113) and PEX14 pair, the binding kinetics of the PEX5 (110-230) and PEX14 may be measured using the AgDNP@Au nanoparticles. Near the first inflection point in the binding curve of PEX5 (110-230) and PEX14, the values found are $k_a$ = 1.24 $\times$ 104 M$^{-1}$s$^{-1}$ and $k_d$ = 2.12 $\times$ 10$^{-3}$ s$^{-1}$. These rates are similar to that of PEX5 (1-113) and PEX14 (see Fig. 27). Therefore, the AgDNP@Au nanoparticles are capable of sensing various protein-protein interactions in the solution phase both qualitatively and quantitatively, and both near equilibrium and kinetically. With these nanoparticles according to the invention, it is possible to detect the binding proteins with each other. For example, for two sets of proteins (TAD-NCBD and PEX5-PEX14), specificity, modularity, and quantification of binding parameters are shown.

Fig. 16 shows different TEM images of AgDNP in post-reaction mixture (Fig. 16a, top) and AgDNP@Au redispersed in 2 mM sodium citrate (Fig. 16b, bottom). Three images are shown for a given sample batch.

Fig. 17 shows a size distribution of AgDNP and AgDNP@Au obtained from manual analysis of >120 particles as shown in Figs. 16a and 16b. The size analysis results in mean sizes of AgDNP and AgDNP@Au of 38.4±4.5 nm (for AgDNP) and 41.0.±4.9 nm (for AgDNP@Au).

Fig. 18a shows STEM-EDX image of a single AgDNP@Au nanoparticle, wherein the silver channel is shown in blue and the gold channel is shown in yellow.

Fig. 18b shows the net intensity of signals of Ag and Au, as a function of position. Position 0 starts at the edge of the rectangle 27 in Fig. 18a, and increases along the arrow 28. The gold coating is approximately 1 nm thick.

Figs. 19a-19d shows extinction spectra of AgDNP and AgDNP@Au as a function of time, and related TEM images.

Fig. 19a shows an extinction spectra of AgDNP redispersed in $H_2O$ as a function of time with an insert showing a zoom-in of the LSPR peak.

Fig. 19b shows a TEM image of AgDNP after redispersion in $H_2O$ for 150 min. The particle tips become rounder and the particle sizes became smaller.

Fig. 19c shows an extinction spectra of AgDNP redispersed in 2 mM sodium citrate and $H_2O$ as a function of time with an insert showing a zoom-in of the LSPR peak.

Fig. 19d shows a TEM image of AgDNP after redispersion in sodium citrate for 150 min. The nanoparticles maintain their decahedral shape.

Fig. 20 shows extinction spectra of AgDNP redispersed in $H_2O$, PBS (pH 7.4), HEPES saline (20 mM HEPES, 150 mM NaCl, pH 7.5), and Tris saline (10 mM Tris, 150 mM NaCl, pH 7.5) after 2 min at room temperature (Fig. 20a).

Figs. 20b-20d show the extinction spectra of AgDNP@Au redispersed in different buffers at room temperature at several time points up to 24 h. The inserts show TEM images of nanoparticles after storage in the corresponding buffer for 24h, then redispersed in $H_2O$.

Fig. 20b shows PBS (pH 7.4), Fig. 20c shwos HEPES saline (20 mM HEPES, 150 mM NaCl, pH 7.5), and Fig. 20d shows Tris saline (10 mM Tris, 150 mM NaCl, pH 7.5)

Fig. 21 shows extinction spectra of AgDNP@Au redispersed in different buffers at physiological temperatures at 37°C for several time points up to 6 h. The inserts show TEM images of nanoparticles after storage in the corresponding buffer at 37°C for 6 h, then redispersed in $H_2O$.

Fig. 21a shows PBS (pH 7.4), Fig. 21b shows HEPES saline (20 mM HEPES, 150 mM NaCl, pH 7.5), and Fig. 21c shows Tris saline (10 mM Tris, 150 mM NaCl, pH 7.5).

Figs. 22a-22b shows EELS data of nanoparticles according to the invention.

Fig. 22a shows data for PEG/NTA-modified AgDNP@Au and Fig. 22b shows data for bare AgDNP@Au, which confirms the appearance of sulfur after surface modification. The elemental composition of the highlighted areas is measured and shows an increase in the sulfur/nitrogen atomic ratio from 0.0044 to 0.129 after PEG/NTA attachment.

Fig. 23 shows a hydrodynamic size distribution by intensity of AgDNP@Au nanoparticles measured using DLS, before and after two-step PEG800/40%NTA-Ni modification. The measured hydrodynamic sizes are 42.5±0.7 nm (before modification) and 45.6±1.2 nm (after modification).

Fig. 24 shows STEM-EDX data of PEG/NTA-Ni-modified AgDNP@Au with an HAADF, Ag, and Au channels.

Fig. 25 shows data related to the colloidal stability of nanoparticles over a range of PEX14:hPEX5 (1-113) concentration ratio from increasing PEX14 concentration. A binding curve of PEX5 (1-113) and PEX14 (16-80) pair, the same as shown in Fig. 15b, in the top left panel (a). Selected spectra, and accompanying zoom-in, at (I) PEX14:PEX5 = 1:1, (II) PEX14:PEX5 = 60, (III) PEX14:PEX5 = 200 are shown. For each measurement, 0.015 μM hPEX5 construct is added to approx. 0.025 nM nanoparticles in a 20 mM sodium phosphate buffer containing 100 mM NaCl and 0.025wt% Tween 20 at pH 7.9. The dilution effect caused by the addition of protein solution is corrected.

Fig. 26 shows data related to the colloidal stability of nanoparticles over a range of PEX14:hPEX5 (110-230) concentration ratio from increasing PEX14 concentration. The binding curve of PEX5 (110-230) and PEX14 (16-80) pair, the same as shown in Fig. 15c, is shown in the top left panel (a). Selected spectra and zoom-ins of peaks for concentration ratios of (I) PEX14:PEX5 = 1:1, (II) PEX14:PEX5 = 10:1, (III) PEX14:PEX5 = 200:1, (IV) PEX14:PEX5 = 1667:1, (V) PEX14:PEX5 = 10000:1 are shown. For each measurement, 0.015 μM hPEX5 construct was added to approx.

0.025 nM nanoparticles in a 20 mM sodium phosphate buffer containing 100 mM NaCl and 0.025wt% Tween 20 at pH 7.9. The dilution effect caused by the addition of protein stock solution was corrected.

Fig. 27 shows a typical kinetic trace of h-PEX5 (110-230) and PEX14 (16-80) binding at a PEX14:PEX5 ratio of 10:1 without pre-mixing (near the first inflection point in Fig. 15c, square "INF"). Obtained binding parameters of a pair of PEX5 (110-230) and PEX14 (16-80) pair are $K_{D1}$ = 171 nM, $k_a$ = 1.24 × 10$^4$ N$^{-1}$s$^{-1}$, $k_d$ = 2.12 × 10$^{-3}$ s$^{-1}$, and $K_{D2}$ = 24.7 $\mu$M. $K_{D1}$ may be used to calculate the binding kinetics parameters. Fitting with an exponential function gives an average $k_{obs}$ = 0.00397 s$^{-1}$ from three measurements. 0.015 $\mu$M His-Tagged PEX5 construct was added to approx. 0.025 nM nanoparticles in a 20 mM sodium phosphate buffer containing 100 mM NaCl and 0.025wt% Tween 20 at pH 7.9.

Fig. 28 shows Hill's coefficients derived from different PEX5-PEX14 binding pairs, with the same data same as shown in Fig. 15.

Fig. 29 shows an extinction spectrum scanned for kinetic measurements. A fit to a Lorentzian function is performed to derive the $\lambda_{max}$ value.

For kinetic measurements, a smaller range of the extinction spectrum ($\pm$ 10 nm from the peak position $\lambda_{max}$) may be scanned at a 0.1 nm interval. A fit to a Lorentzian function may be performed to determine the $\lambda_{max}$ value. Fig. 29 shows a representative spectrum and fit, along with fit residuals (bottom). Before the addition of the 2nd protein, at least five consecutive spectra are measured to determine the starting peak position. Once the 2nd protein is added, the sample is measured 20-30 s/scan for 25 minutes. After the kinetics measurement, the entire spectrum (350-750 nm, 0.2 nm interval) is scanned again to obtain the "final" spectrum.

The observable binding rate $k_{obs}$ is obtained from an exponential fit of the time-dependent change in the spectral peak $\lambda_{max}$. The association rate constant $k_a$ and dissociation rate constant $k_d$ may be derived from the equation $k_{obs}$ = [P] · $k_a$ + $k_d$ and the equilibrium dissociation constant $K_D$ = $k_d$ / $k_a$. [P] is the concentration of the 2nd protein and $K_D$ is determined from the concentration-dependent "binding curve", as it equals the solution concentration of the 2nd protein at which 50% binding is reached. Each binding curve around an inflection point is fitted with the Hill-Waud equation:

$$\Delta\lambda_{LSPR} = \frac{\Delta\lambda_m [P]^n}{K_h^n + [P]^n} + offset$$

where $K_h$ is the apparent dissociation constant, $\Delta\lambda_m$ is the maximum peak shift of $\Delta\lambda$LSPR of the 100% bound state, and n is the Hill (cooperativity) coefficient. An offset was only applied when analyzing the second inflection point of a concentration series "binding curve".

By introducing the binding fraction $\theta$ = $\Delta\lambda$LSPR/$\Delta\lambda$m, the Hill's coefficient can be derived from the slope of the linear fitting of log($\theta$/(1-$\theta$)) versus log [P] as follows:

$$\log\left(\frac{\theta}{1-\theta}\right) = n\log[P] - n\log[K_h]$$

An estimation of AgDNP@Au particle concentration can be made assuming a full conversion of AgNO$_3$ to AgDNP in the AgDNP synthesis and a shape of the AgDNP being a pentagonal bipyramid with all faces being equilateral triangles with an edge length $L_{AgDNP}$ 25.0 nm according to TEM images (see Fig. 1b). The AgDNP concentration $c_{AgDNP}$ is thus 0.235 nM. For the synthesis of each AgDNP@Au batch, 3 mL HAuCl$_4$ was slowly added to 3 mL freshly prepared AgDNP reaction mixture. This means that the concentration of nanoparticles is diluted by half. Hence, the concentration of AgDNP@Au is 0.117 nM.

## Claims

1. A nanoparticle (1) having core (2), a first surface functionalization (4), and a second surface functionalization (5), wherein

    the core (2) comprises or consists of a metal, preferably silver, and
    wherein an inorganic coating (3) is arranged around the core (2), the coating (3) preferably comprising or consisting of gold, and
    wherein the first surface functionalization (4) is adapted to provide colloidal stability of the nanoparticle (1) in, preferably aqueous, solvents (19) and substantially prevents non-specific binding of macromolecules (55), in particular macromolecules including proteins, DNA, and/or RNA, and
    wherein the second surface functionalization (5) includes one of a binding site for a binding partner, preferably a binding site for a poly-histidine tag of a biomolecule (55), and a biomolecule, preferably a protein.

2. The nanoparticle according to claim 1, wherein the nanoparticle (1) has a characteristic size between 20 nm and 60 nm, preferably between 30 nm and 45 nm.

3. The nanoparticle according to any one of claims 1 or 2, wherein the nanoparticle (1) has a shape comprising or consisting of flat facets (7) delimited by edges (8) which meet in a tip (6), preferably a substantially decahedral shape, particularly preferably a pentagonal bipyramid.

4. The nanoparticle (1) according to any one of the preceding claims, wherein the first surface functionalization (4) comprises polyethylene glycol or a derivative thereof, preferably formed by a first ligand, preferably wherein the first ligand has molecular weight of less than 1.5 kDa, particularly preferably less than 1 kDa.

5. The nanoparticle (1) according to claim 5, wherein the first ligand (4) is a thiolated polyethylene glycol or a derivative thereof, particularly preferably methoxy polyethylene glycol thiol.

6. The nanoparticle (1) according to any one of the preceding claims, wherein the second surface functionalization (5) includes nitriloacetic acid chelated with a metal ion, preferably Ni(II) and/or Co(II), preferably formed by a second ligand.

7. The nanoparticle (1) according to claim 6, wherein the second ligand (5) is thiolated alkane-polyethylene glycol-nitriloacetic acid chelated with Ni(II), preferably methoxy polyethylene glycol thiol nitriloacetic acid chelated with Ni(II).

8. A method of functionalizing colloidal nanoparticles (1), comprising

providing nanoparticles (1), preferably colloidal nanoparticles (1) with a gold coating (3), particularly preferably decahedral silver nanoparticles with a gold coating (3), suspending the colloidal nanoparticles (1) in a solvent, preferably one of dimethyl sulfoxide, water, aqueous biological buffer, aqueous buffered saline, blood serum, and cell extracts,
adding a ligand mixture (9), preferably a ligand mixture containing methoxy polyethylene glycol thiol and nitriloacetic acid, and preferably incubating the nanoparticles, and
subsequently, washing the nanoparticles at least once, preferably twice.

9. The method of claim 8, further comprising the steps of, after washing and preferably redispersing,

incubating the nanoparticles (1) in a solution (10) comprising a metal salt, preferably $NiCl_2$, and
subsequently, a second step of washing the nanoparticles (1), at least once.

10. A nanoparticle functionalized by the method of any one of claim 8 or 9.

11. A suspension (20) comprising colloidal particles (1), preferably a plurality of nanoparticles (1) according to any one of claims 1 to 7, or 10, and a solvent (19), preferably an aqueous biological buffer, wherein the colloidal particles (1) are suspended in the solvent (19).

12. The suspension according to claim 11, wherein the concentration of the colloidal particles (1) in the solvent is between 0.01 nM and 20 nM, preferably between 0.05 and 0.2 nM.

13. A method of determining a value representing, preferably quantifying, an interaction between a first and a second molecule, comprising

a.) providing a first suspension (20) according to any one of claims 11 to 12,
b.) optionally, adding a first molecule, preferably a protein, to the first suspension, wherein the first molecule has a tag for binding specifically to the binding site, such as to immobilize the first molecule to the binding site on the colloidal particles (1),
c.) preferably after adding the first molecule, adding a second molecule, preferably a second biomolecule, preferably a protein, to the first suspension, resulting in a first concentration,
d.) obtaining at least a first optical value after addition of the second molecule, and
e.) obtaining a change of the optical value to a reference value, preferably a reference value obtained after step b.) and before step c.).

14. The method according to claim 13, wherein the step of measuring said first optical value includes measuring an optical extinction value, preferably recording an extinction or absorption spectrum using a UV/VIS spectrophotometer or other liquid-phase spectroscopy technique, of the suspension (20), and

and wherein the first optical value is a position of an absorption peak corresponding to a localized surface plasmon resonance of the colloidal particles.

15. The method according to any one of claims 13 or 14, optionally wherein steps a.) and b.) are repeated to obtain second suspension,

step c.) is repeated resulting in a second concentration different from the first concentration,
repeating steps d.) and e.) to obtain a second optical value
calculating an equilibrium dissociation constant from a concentration dependence in the first and second change.

16. The method according to any one of claims 13 to 15, wherein step c.) is repeated at least once, preferably a plurality of times until the optical value remains constant between two measurements, and calculating a kinetic constant, preferably an association or a dissociation constant from the time dependency of the change.

Fig. 1a

Fig. 1b

Fig. 2a

Fig. 2b

Fig. 2c

Fig. 3

Fig. 4a

Fig. 4b

Fig. 5a

Fig. 5b

Fig. 6

Fig. 7

Fig. 8

Fig. 9a          Fig. 9b          Fig. 9c

Fig. 9d          Fig. 9e          Fig. 9f

Fig. 10a          Fig. 10b          Fig. 10c

Fig. 11

Fig. 12a

Fig 12b

Fig. 12c

Fig. 13

Fig. 14

Fig. 15

Fig. 16

Fig. 17

Fig. 18a

Fig. 18b

Fig. 19

Fig. 20

Fig. 21

Fig. 22

Fig. 23

Fig. 24

Fig. 25

Fig. 26

Fig. 27

Fig. 28

Fig. 29

Europäisches
Patentamt

European
Patent Office

Office européen
des brevets

# EUROPEAN SEARCH REPORT

Application Number

EP 23 21 4385

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/047804 A1 (MEHRA RAJESH K [US] ET AL) 18 February 2016 (2016-02-18) | 1-3, 10-12 | INV. G01N33/543 |
| Y | * paragraphs [0037], [0071], [0087]-[0092] * | 4-9, 13-16 | G01N33/553 G01N33/557 |
| | & JUI-TING TAI: "Protein-Silver Nanoparticle Interactions to Colloidal Stability in Acidic Environments", LANGMUIR, vol. 30, no. 43, 21 October 2014 (2014-10-21), pages 12755-12764, XP093160784, US ISSN: 0743-7463, DOI: 10.1021/la5033465 * abstract * | | |
| Y | KAMIL RAHME: "Highly stable PEGylated gold nanoparticles in water: applications in biology and catalysis", RSC ADVANCES, vol. 3, no. 43, 1 January 2013 (2013-01-01), page 21016, XP093160952, GB ISSN: 2046-2069, DOI: 10.1039/c3ra41873a * abstract; page 2106, right-column, line 13 - page 21017, left-column, line 2; page 21017, right-column, section "grafting of poly(ethylene glycol) ligands"; page 21020, right-column, 2nd par. - page 21022, left-column * | 4,5 | |

TECHNICAL FIELDS SEARCHED (IPC)

G01N

-/--

The present search report has been drawn up for all claims

| Place of search | Date of completion of the search | Examiner |
|---|---|---|
| Munich | 10 May 2024 | Lindberg, Pia |

EPO FORM 1503 03.82 (P04C01)

page 1 of 3

Europäisches
Patentamt

European
Patent Office

Office européen
des brevets

**EUROPEAN SEARCH REPORT**

**Application Number**

**EP 23 21 4385**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JOSHUA D. SWARTZ: "Development of a Histidine-Targeted Spectrophotometric Sensor Using Ni(II)NTA-Functionalized Au and Ag Nanoparticles", LANGMUIR, vol. 27, no. 24, 20 December 2011 (2011-12-20), pages 15330-15339, XP093160937, US ISSN: 0743-7463, DOI: 10.1021/la202937j Retrieved from the Internet: URL:https://cdn.vanderbilt.edu/vu-my/wp-content/uploads/sites/1554/2014/09/14124108/la202937j.pdf> * abstract; page 15331, right-column, 1st full par.; page 15332; bridging paragraph on page 15335, right-column -15335, left-column; page 15336, right-column, last par. - page 15337, left-column; figure 2 * ----- | 6-9 | |
| Y | SIGAL G B ET AL: "SELF-ASSEMBLED MONOLAYER FOR THE BINDING AND STUDY OF HISTIDINE-TAGGED PROTEINS BY SURFACE PLASMON RESONANCE", ANALYTICAL CHEMISTRY, AMERICAN CHEMICAL SOCIETY, US, vol. 68, no. 3, 1 January 1996 (1996-01-01), pages 490-497, XP000916112, ISSN: 0003-2700, DOI: 10.1021/AC9504023 * abstract; page 494, right-column, 2nd - page 497. * ----- -/-- | 13-16 | TECHNICAL FIELDS SEARCHED (IPC) |

The present search report has been drawn up for all claims

| Place of search | Date of completion of the search | Examiner |
|---|---|---|
| Munich | 10 May 2024 | Lindberg, Pia |

EPO FORM 1503 03.82 (P04C01)

**EUROPEAN SEARCH REPORT**

Application Number

EP 23 21 4385

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FLESCH JULIA ET AL: "Self-assembly of robust gold nanoparticle monolayer architectures for quantitative protein interaction analysis by LSPR spectroscopy", ANALYTICAL AND BIOANALYTICAL CHEMISTRY, SPRINGER BERLIN HEIDELBERG, BERLIN/HEIDELBERG, vol. 412, no. 14, 21 March 2020 (2020-03-21), pages 3413-3422, XP037114674, ISSN: 1618-2642, DOI: 10.1007/S00216-020-02551-6 [retrieved on 2020-03-21] * abstract; page 3415, right-column, last par. – page 3416, left-column; page 3417, left-column, 2nd par. – page 3420, left-column, 1st par. * | 13-16 | |
| A | JUI-CHANG CHEN: "Physical Properties and the Reconstruction of Unstable Decahedral Silver Nanoparticles Synthesized Using Plasmon-Mediated Photochemical Process", NANOMATERIALS, vol. 12, no. 7, 24 March 2022 (2022-03-24), page 1062, XP093161109, ISSN: 2079-4991, DOI: 10.3390/nano12071062 * the whole document * | 1-16 | TECHNICAL FIELDS SEARCHED (IPC) |

The present search report has been drawn up for all claims

| Place of search | Date of completion of the search | Examiner |
|---|---|---|
| Munich | 10 May 2024 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 23 21 4385

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2024

| Patent document cited in search report | Publication date | Patent family member(s) | Publication date |
|---|---|---|---|
| US 2016047804 A1 | 18-02-2016 | AU 2015301632 A1 | 02-03-2017 |
| | | AU 2021202253 A1 | 06-05-2021 |
| | | AU 2023270256 A1 | 07-12-2023 |
| | | BR 112017002603 A2 | 17-07-2018 |
| | | CA 2957134 A1 | 18-02-2016 |
| | | CA 3209879 A1 | 18-02-2016 |
| | | CN 106796228 A | 31-05-2017 |
| | | CN 112415192 A | 26-02-2021 |
| | | DK 3180619 T3 | 13-09-2021 |
| | | EP 3180619 A2 | 21-06-2017 |
| | | EP 3943943 A1 | 26-01-2022 |
| | | ES 2888230 T3 | 03-01-2022 |
| | | HU E056514 T2 | 28-02-2022 |
| | | IL 280263 A | 01-03-2021 |
| | | IL 291833 A | 01-06-2022 |
| | | IL 300266 A | 01-03-2023 |
| | | JP 6945445 B2 | 06-10-2021 |
| | | JP 7240460 B2 | 15-03-2023 |
| | | JP 2017524140 A | 24-08-2017 |
| | | JP 2022008384 A | 13-01-2022 |
| | | JP 2023067953 A | 16-05-2023 |
| | | KR 20170040331 A | 12-04-2017 |
| | | KR 20230025925 A | 23-02-2023 |
| | | PL 3180619 T3 | 13-12-2021 |
| | | PT 3180619 T | 20-09-2021 |
| | | SG 10201901240T A | 28-03-2019 |
| | | SG 11201700864W A | 30-03-2017 |
| | | TW 201614219 A | 16-04-2016 |
| | | US 2016047804 A1 | 18-02-2016 |
| | | US 2020150115 A1 | 14-05-2020 |
| | | US 2022074934 A1 | 10-03-2022 |
| | | WO 2016025703 A2 | 18-02-2016 |
| | | ZA 201700873 B | 24-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2014106469 A1
- US 11280784 B2
- WO 20222014611 A1

### Non-patent literature cited in the description

- **FLESCH et al.** *Anal. Bioanal. Chem.*, 2020, vol. 412, 3413-3422
- **HOBBS et al.** *Chem. Commun.*, 2016, vol. 52, 9785-9788
- **MURSHID, N.** ; **KEOGH, D.** ; **KITAEV, V.** Optimized Synthetic Protocols for Preparation of Versatile Plasmonic Platform Based on Silver Nanoparticles with Pentagonal Symmetries. *Part. Part. Syst. Charact.*, 2014, vol. 31 (2), 178-189
- **MUSHID, N.** ; **GOUREVICH, I** ; **COOMBS, N.** ; **KITAEV, V.** Gold Plating of Silver Nanoparticles for Superior Stability and Preserved Plasmonic and Sensing Properties. *Chem. Commun.*, 2013, vol. 49 (97), 11355-11357
- *J. Am. Chem. Soc.*, 2012, vol. 134 (13), 6000-6005
- *Langmuir*, 2014, vol. 30 (7), 1864-1870